# EUROPEAN PATENT SPECIFICATION

(11) **EP 2 598 502 B1**
(45) Date of publication and mention of the grant of the patent: **14.05.2014**
(21) Application number: 12762305.6
(22) Date of filing: 26.09.2012
(51) Int. Cl.: C07D 417/12, A61K 31/4402, A61P 31/00, A61P 31/22

(54) **N-[5-(AMINOSULFONYL)-4-METHYL-1,3-THIAZOL-2-YL]-N-METHYL-2-[4-(2-PYRIDINYL)PHENYL]ACETAMIDE MESYLATE MONOHYDRATE HAVING A SPECIFIC PARTICLE SIZE DISTRIBUTION RANGE AND A SPECIFIC SURFACE AREA RANGE FOR USE IN PHARMACEUTICAL FORMULATIONS.**
N-[5-(AMINOSULFONYL)-4-METHYL-1,3-THIAZOL-2-YL]-N-METHYL-2-[4-(2-PYRIDINYL)PHENYL]ACETAMIDMESYLAT-MONOHYDRAT MIT EINEM SPEZIFISCHEN TEILCHENGRÖSSENVERTEILUNGSBEREICH UND EINEM SPEZIFISCHEN OBERFLÄCHENBEREICH
MONOHYDRATE DE MESYLATE ACÉTAMIDE N-[5-(AMINOSULFONYL)-4MÉTHYL-1,3-THIAZOL-2-YL]-N-MÉTHYL-2-[4-(2-PYRIDINYL)PHÉNYL] DISPOSANT D'UNE GAMME DE DISTRIBUTION DE TAILLE DE PARTICULES SPÉCIFIQUES ET PORTÉE DE SURFACE SPÉCIFIQUE

(30) Priority: 26.09.2011 WO PCT/EP2011/007803
(43) Date of publication of application: 05.06.2013
(73) Proprietor: AiCuris GmbH & Co. KG, 42117 Wuppertal (DE)
(72) Inventor: Schwab, Wilfried, 42551 Velbert (DE); Birkmann, Alexander, 42327 Wuppertal (DE); Paulus, Kerstin, 40882 Ratingen (DE); Vögtli, Kurt, 5273 Oberhofen AG (DE); Haag, Dieter, 4433 Ramlinsburg BL (DE); Mass, Stephan, 47803 Krefeld (DE); Ruepp, Kristian, 50823 Köln (DE)
(74) Representative: Arth, Hans-Lothar
(86) International application number: PCT/EP2012/068958
(87) International publication number: WO 2013/045491

(56) References cited:
- WO-A1-01/47904
- WO-A1-2006/103011
- U. A. K. BETZ ET AL: "Potent In Vivo Antiviral Activity of the Herpes Simplex Virus Primase-Helicase Inhibitor BAY 57-1293", ANTIMICROBIAL AGENTS AND CHEMOTHERAPY, vol. 46, no. 6, 1 June 2002 (2002-06-01), pages 1766-1772, XP055012766, ISSN: 0066-4804, DOI: 10.1128/AAC.46.6.1766-1772.2002

## Description

The present invention relates to the crystalline mono mesylate monohydrate salt of N-[5-(aminosulfonyl)-4-methyl-1,3-thiazol-2-yl]-N-methyl-2-[4-(2-pyridinyl)phenyl]-acetamide in a definite particle size distribution and a specific surface area range, which has demonstrated increased long term stability and release kinetics from pharmaceutical compositions, as well as to pharmaceutical compositions containing said N-[5-(aminosulfonyl)-4-methyl-1,3-thiazol-2-yl]-N-methyl-2-[4-(2-pyridinyl)phenyl]acetamide mono mesylate monohydrate having the afore-mentioned particle size distribution and specific surface area range.

### Background of the invention

Synthesis of N-[5-(aminosulfonyl)-4-methyl-1,3-thiazol-2-yl]-N-methyl-2-[4-(2-pyridinyl)phenyl]acetamide is known from EP 1244641 B1, and the use of acidic components including methanesulfonic acid for the formulation of tablets containing micronized N-[5-(aminosulfonyl)-4-methyl-1,3-thiazol-2-yl]-N-methyl-2-[4-(2-pyridinyl)phenyl]acetamide is disclosed by WO 2006/103011 A1. It is also disclosed that N-[5-(aminosulfonyl)-4-methyl-1,3-thiazol-2-yl]-N-methyl-2-[4-(2-pyridinyl)phenyl]acetamide is active in parenteral, oral and topical formulations (U. A. K. Betz et al., Antimicrob. Agents Chemother. 2002, 46(6), 1766-1772).

The EP 11007823 A1 is the Applicant's previous European patent application related with the present invention and directed to an improved synthesis of N-[5-(aminosulfonyl)-4-methyl-1,3-thiazol-2-yl]-N-methyl-2-[4-(2-pyridinyl)phenyl] acetamide and the mesylate monohydrate salt thereof by using boronic acid derivatives or borolane reagents while avoiding toxic organic tin compounds and to the mesylate monohydrate salt of N-[5-(aminosulfonyl)-4-methyl-1,3-thiazol-2-yl]-N-methyl-2-[4-(2-pyridinyl)phenyl]acet-amide.

It is the objective of the present invention to provide a specific form of a salt of the compound N-[5-(aminosulfonyl)-4-methyl-1,3-thiazol-2-yl]-N-methyl-2-[4-(2-pyridinyl)phenyl]acetamide which exhibits improved properties in regard to stability and bioavailability and thus makes this specific form of the salt preferable for the manufacturing of pharmaceutical compositions and to provide pharmaceutical compositions containing such a specific salt so that these pharmaceutical compositions exhibit improved properties in regard to stability and bioavailability of the contained specific form of a salt of the compound N-[5-(aminosulfonyl)-4-methyl-1,3-thiazol-2-yl]-N-methyl-2-[4-(2-pyridinyl)phenyl]acetamide.

The objective of the present invention is solved by the teaching of the independent claims. Further advantageous features, aspects, and details of the invention are evident from the dependent claims, the description, the figures, and the examples of the present application.

### Description of the invention

At room temperature, there are four polymorphic forms and an amorphous form of the free base of N-[5-(aminosulfonyl)-4-methyl-1,3-thiazol-2-yl]-N-methyl-2-[4-(2-pyridinyl)-phenyl]acetamide. In addition, several solvates can be detected for the free base of N-[5-(aminosulfonyl)-4-methyl-1,3-thiazol-2-yl]-N-methyl-2-[4-(2-pyridinyl)-phenyl]acetamide depending on the solvent. Based on the existence of the afore-mentioned forms of the free base of N-[5-(aminosulfonyl)-4-methyl-1,3-thiazol-2-yl]-N-methyl-2-[4-(2-pyridinyl)-phenyl]acetamide at room temperature, no conclusion on thermic stability can be drawn. Hence, the free base of N-[5-(aminosulfonyl)-4-methyl-1,3-thiazol-2-yl]-N-methyl-2-[4-(2-pyridinyl)-phenyl]acetamide is not suitable for a long-term stable formulation, because long-term properties of the free base of N-[5-(aminosulfonyl)-4-methyl-1,3-thiazol-2-yl]-N-methyl-2-[4-(2-pyridinyl)-phenyl]acetamide cannot be determined. In addition, the low solubility is responsible for unfavourable drug release and resorption properties compared to the mesylate monohydrate salt.

Surprisingly it was found that the mesylate monohydrate salt of N-[5-(aminosulfonyl)-4-methyl-1,3-thiazol-2-yl]-N-methyl-2-[4-(2-pyridinyl)phenyl]acetamide overcomes all drawbacks of the free base or other salts if that mesylate monohydrate salt has a specific PSD (particle size distribution), PSR (particle size range) and SSA (specific surface area).

Thus the present invention relates to crystalline N-[5-(aminosulfonyl)-4-methyl-1,3-thiazol-2-yl]-N-methyl-2-[4-(2-pyridinyl)-phenyl]acetamide mono methanesulfonic acid monohydrate of the following formula 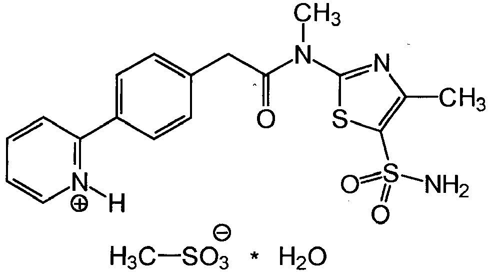 wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a particle size range from 1 to 500 µm, a particle size distribution which is defined by d(0.1) from 2 to 100 µm, d(0.5) from 30 to 210 µm and d(0.9) from 70 to 400 µm and a specific surface area of less than 1.0 m²/g.

The synthesis of N-[5-(aminosulfonyl)-4-methyl-1,3-thiazol-2-yl]-N-methyl-2-[4-(2-pyridinyl)phenyl]acetamide mono methanesulfonic acid monohydrate is shown below

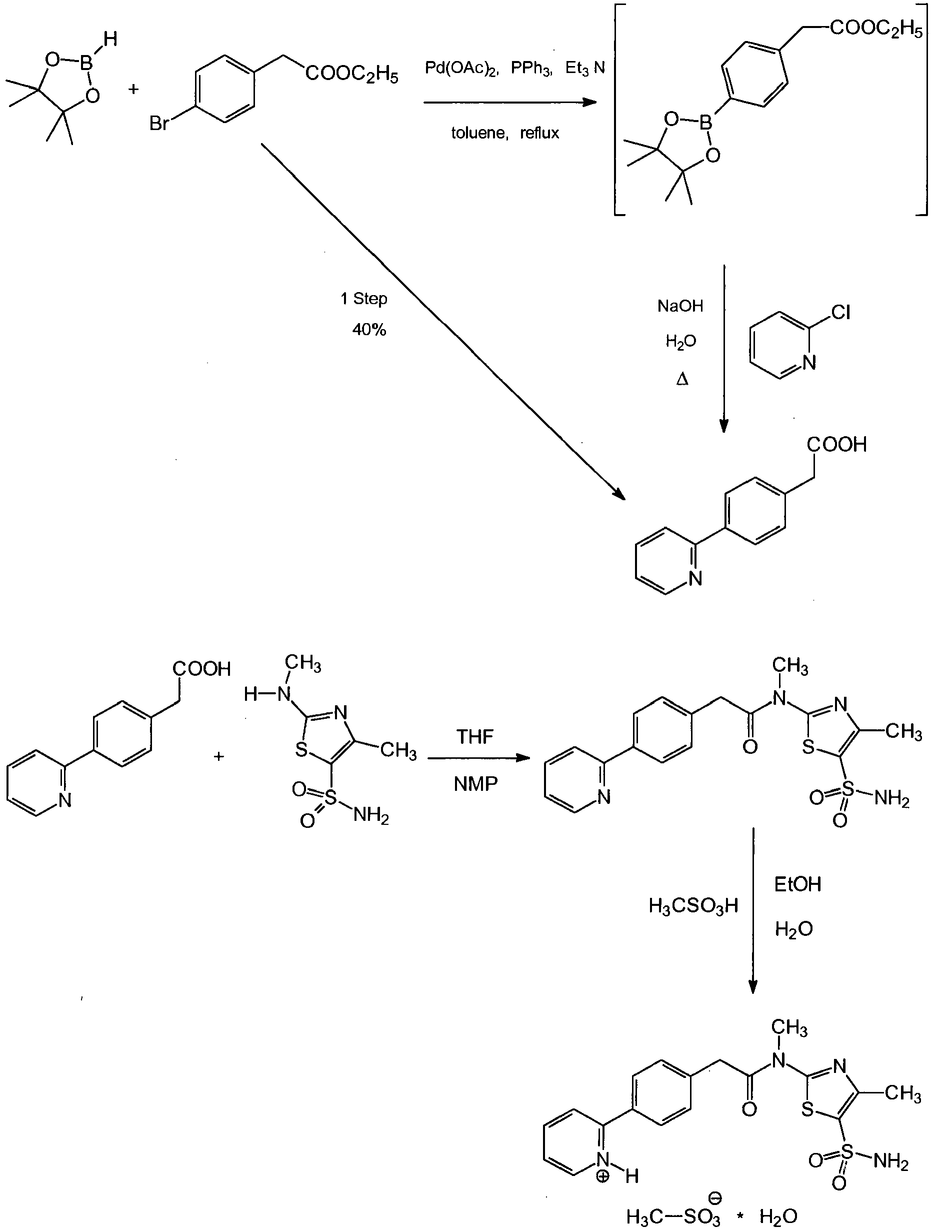

The above synthesis uses a boronic acid derivative, a borolane or a borinane reagent which is reacted with ethyl-4-bromophenylacetate in order to obtain the key intermediate (4-pyridin-2-ylphenyl) acetic acid in a single stage with an over all yield of about 40% of theory. The (4-pyridin-2-ylphenyl)acetic acid is reacted with 4-methyl-2-(methylamino)-1,3-thiazole-5-sulfonamide to the N-[5-(aminosulfonyl)-4-methyl-1,3-thiazol-2-yl]-N-methyl-2-[4-(2-pyridinyl)phenyl]acetamide (which is also named as "free base" herein) which is then converted to the definite mono mesylate monohydrate salt (also named herein as "mesylate monohydrate" or "mesylate monohydrate salt") exhibiting a specific PSD, PSR and SSA. The single reaction steps for the synthesis of the N-[5-(aminosulfonyl)-4-methyl-1,3-thiazol-2-yl]-N-methyl-2-[4-(2-pyridinyl)phenyl]acetamide mesylate monohydrate are summarized below.

### Step A: Reacting compound A of the following general formula A*

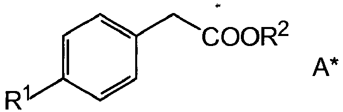

wherein
R¹ represents a leaving group and
R² represents an alkyl residue with 1 to 6 carbon atoms or a cycloalkyl residue with 3 to 6 carbon atoms,
with a boronic acid derivative, borolane, borinane or diboronic acid reagent under elimination of R¹-H or R¹-B(OR)₂ and formation of an intermediate boronic acid derivative of compound A,
wherein preferred catalysts for the reaction are the reagent systems palladium acetate with triethylamine and with triphenylphosphine or PdCl₂(PPh₃)₂ with triethylamine,
wherein the intermediate boronic acid derivative is then reacted with the pyridine compound B of the following general formula B* 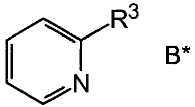 wherein
R³ represents a leaving group
under basic conditions in order to obtain the (4-pyridin-2-ylphenyl)acetic acid as an alkaline solution of the corresponding carboxylate salt.

The resulting (4-pyridin-2-ylphenyl)acetic acid was purified by simple washings at different pH and filtration steps followed by precipitation or crystallization, preferably by properly adjusting the pH of an aqueous acidic solution of (4-pyridin-2-ylphenyl)acetic acid with an appropriate amount of base to 3.5 - 5.0, preferably 3.8 - 4.7. Beside the simple washing and filtration step, no further purification of the (4-pyridin-2-ylphenyl)acetic acid or any of the intermediates by, for instance, recrystallization or chromatography is required.

### Step B: Reacting (4-pyridin-2-ylphenyl)acetic acid obtained from step A with 4-methyl-2-(methylamino)-1,3-thiazole-5-sulfonamide

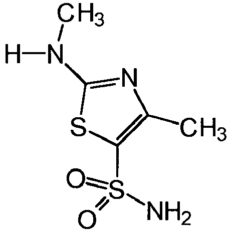

in order to obtain N-[5-(aminosulfonyl)-4-methyl-1,3-thiazol-2-yl]-N-methyl-2-[4-(2-pyridinyl)phenyl]acetamide of the formula

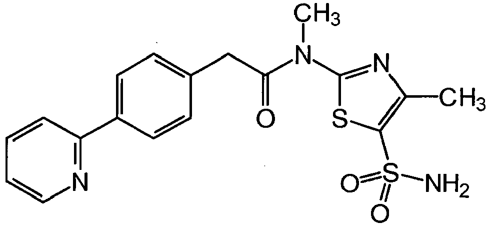

The N-[5-(aminosulfonyl)-4-methyl-1,3-thiazol-2-yl]-N-methyl-2-[4-(2-pyridinyl)-phenyl]acetamide is thereafter most preferably converted (as step C) to the so far unknown monohydrate of the mesylate salt of N-[5-(aminosulfonyl)-4-methyl-1,3-thiazol-2-yl]-N-methyl-2-[4-(2-pyridinyl)phenyl]acetamide. The obtained N-[5-(amino-sulfonyl)-4-methyl-1,3-thiazol-2-yl]-N-methyl-2-[4-(2-pyridinyl)phenyl]acetamide mesylate monohydrate is then formulated as particles having a particle size in the range from 1 µm to 500 µm and a particle size distribution defined by d(0.1) from 2 to 100 µm, d(0.5) from 30 to 210 µm and d(0.9) from 70 to 400 µm and a specific surface area of the particles less than 1.0 m²/g, and more preferably a PSD defined by d(0.1) from 10 to 75 µm, d(0.5) from 100 to 175 µm, d(0.9) from 200 to 350 µm with a SSA of the particles less than 0.3 m²/g.

In the above synthesis, the term "leaving group" as used herein is a molecular fragment that departs with a pair of electrons in heterolytic bond cleavage. Leaving groups can be anions or neutral molecules. Common anionic leaving groups are halides such as Cl⁻, Br⁻, and I⁻, and sulfonate esters, such as *para*toluenesulfonate ("tosylate", TsO⁻), trifluoromethanesulfonate ("triflate", TfO⁻, CF₃SO₂O⁻), benzenesulfonate ("besylate, C₆H₅SO₂O⁻) or methanesulfonate ("mesylate", MsO⁻).

General formula A* as shown below 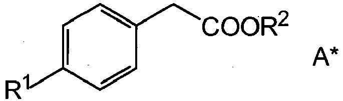 covers all phenyl acetic acid esters having a leaving group on the phenyl residue in position 4.

Thus R¹ preferably represents -F, -Cl, -Br, -I, -OMs, -OTf and -OTs. The group "-OMs" refers to -OMesylate, the group "-OTf' refers to -OTriflate and the group "-OTs" refers to -OTosylate.

The group R² represents an alkyl residue with 1 to 6 carbon atoms or a cycloalkyl residue with 3 to 6 carbon atoms, and preferably -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, cyclo-C₃H₅, cyclo-C₄H₇, cyclo-C₅H₉, cyclo-C₆H₁₁. More preferred are -CH₃, -C₂H₅, -C₃H₇, -CH(CH₃)₂, -C₄H₉, -CH₂-CH(CH₃)₂, -CH(CH₃)-C₂H₅, -C(CH₃)₃, and -C₅H₁₁. Especially preferred are -CH₃, -C₂H₅, -C₃H₇, and -CH(CH₃)₂.

Various boronic acid derivatives, borolanes and borinanes as well as the corresponding diboronic acid derivatives can be used in step A of the inventive synthesis disclosed herein. Preferred are borolanes of the following general formula: 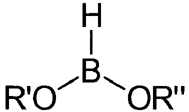 wherein R' and R" are independently of each other any substituted or unsubstituted, linear or branched alkyl group with 1 to 10 carbon atoms or cycloalkyl group with 3 to 10 carbon atoms, or R' and R" can also form together with the boron atom a heterocyclic ring wherein R' and R" together form a substituted or unsubstituted, linear or branched alkylen group with 2 to 10 carbon atoms. Preferably R' and R" represent independently of each other -CH₃, -C₂H₅, -C₃H₇, -CH(CH₃)₂, -C₄H₉, -CH₂-CH(CH₃)₂, -CH(CH₃)-C₂H₅, -C(CH₃)₃, and -C₅H₁₁. The cyclic borolanes are preferred.

The following borolanes, borinanes and diboronic acid derivatives are preferred:

| | | |
|---|---|---|
| 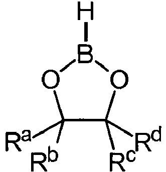 | 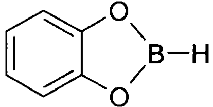 | 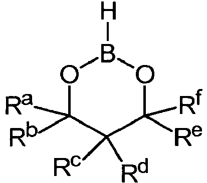 |
| Borolane | Catecholboran | Borinane |
| 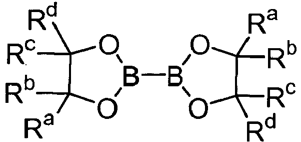 | | 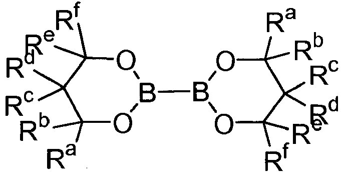 |
| Diborolane | | Diborinane |

wherein R^{a}, R^{b}, R^{c}, R^{d}, R^{e} and R^{f} represent independently of each other a substituted or unsubstituted, linear or branched alkyl group with 1 to 10 carbon atoms or cycloalkyl group with 3 to 10 carbon atoms. Preferred are the linear alkyl residues with 1 to 6 carbon atoms, and most preferred are -CH₃, -C₂H₅, -C₃H₇ and -CH(CH₃)₂.

Especially preferred examples for the above borone containing compounds are 4,4,5,5-tetramethyl[1,3,2]dioxaborolane (pinacolborane), [1,3,2]dioxaborolane, [1,3,2]dioxaborinane, 5,5-dimethyl[1,3,2]dioxaborinane, 4,6,6-trimethyl[1,3,2]-dioxaborinane, 4,4,6,6-tetramethyl[1,3,2]-dioxaborinane, 4,4,5,5,6,6-hexamethyl[1,3,2]-dioxaborinane, diisopropoxyborane, hexahydrobenzo[1,3,2]dioxaborole, 9,9-dimethyl-3,5-dioxa-4-bora-tricyclo-[6.1.1.6^{2,6}]decane, 6,9,9-trimethyl-3,5-dioxa-4-bora-tricyclo[6.1.1.6^{2,6}]decane, B2Pin2 (bis(pinacolato)diborane), bis(nepentyl glycolato)diboron and catecholboran.

In step A this boronic acid derivative, borolane, borinane or diboronic acid reagent is reacted with a compound A of general formula A* in order to obtain an intermediate borolan or borinane reagent which is not isolated and purified. This reaction may be supported by the use of either catalysts prepared in situ by combination of palladium salts such as [Pd(OAc)₂] and PdCl₂ with triphenylphosphine (PPh₃), tri-ortho-tolylphosphine (P(o-Tol)₃), tricyclohexylphosphine (PCy₃), tri-tert.-butylphosphine, 1,4-Bis-(diphenylphosphino)-butane (dppb), and 1,1'-Bis-(diphenylphosphino)-ferrocene dppf or preformed catalysts such as Pd(PPh₃)₂Cl₂, Pd(PPh₃)₄, Fibrecat 1032, and Pd(dppf)Cl₂ in the presence of a variety of organic and inorganic bases such as triethylamine (Et₃N), NaOAc, KOAc, and K₃PO₄. For this reaction heating to temperature between 70°C and 150°C, preferably between 80°C and 130°C, more preferably between 90°C and 110°C is used. Moreover aprotic and preferably apolar solvents and preferably aromatic solvents such as benzene or toluene or xylenes are used.

The intermediate boronic acid reagent is subsequently reacted with a pyridinyl compound of the general formula B*, wherein R³ represents a leaving group. Thus R³ represents -F, -Cl, -Br, -I, -OMs, -OTf and -OTs and preferably -Cl or -Br.

The corresponding (4-pyridin-2-ylphenyl)acetic acid ester is in situ treated with a aqueous base in order to cleave the ester linkage. It could be advantageous to heat the reaction mixture during the coupling / saponification step to moderate temperature and preferably to temperature between 40°C and 90°C, more preferably between 45°C and 80°C, still more preferably between 50°C and 70°C and most preferably between 55°C and 65°C.

After purification and isolation of the key intermediate (4-pyridin-2-ylphenyl)acetic acid, the (4-pyridin-2-ylphenyl)acetic acid was obtained in a yield of about 40% of theory including only one isolation and purification step.

Thereafter the (4-pyridin-2-ylphenyl)acetic acid was reacted with 4-methyl-2-(methylamino)-1,3-thiazole-5-sulfonamide of the formula 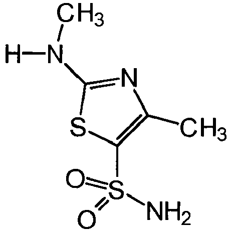 which was prepared according to the synthesis disclosed in EP 1244641 B1 in order to obtain N-[5-(aminosulfonyl)-4-methyl-1,3-thiazol-2-yl]-N-methyl-2-[4-(2-pyridinyl)phenyl]acetamide of the formula

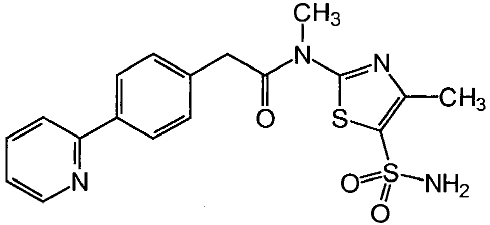

This N-[5-(aminosulfonyl)-4-methyl-1,3-thiazol-2-yl]-N-methyl-2-[4-(2-pyridinyl)-phenyl]acetamide was thereafter converted to the crystalline mesylate monohydrate salt, wherein the typical particle size range is from 1 to 500 µm and the particle size distribution is preferably defined by d(0.1) from 2 to 100 µm, d(0.5) from 30 to 210 µm and d(0.9) from 70 to 400 µm and wherein the crystalline particles have a specific surface area of less than 1.0 m²/g, and more preferably the PSD is defined by d(0.1) from 10 to 75 µm, d(0.5) from 100 to 175 µm, d(0.9) from 200 to 350 µm and the SSA of the particles is less than 0.3 m²/g.

The parameter d(0.1) refers to the mesh size of a single notional sieve allowing 10% of the total of all particles of the sample to pass. Thus d(0.1) = 2 - 100 µm means that the upper limit of the particle size range defining the 10% of smallest particles in the sample is between 2 µm to 100 µm. Thus 10% of the total particles have a particle size of not more than d(0.1) meaning in this case that they have a maximum size of 2 µm to 100 µm.

Accordingly the parameter d(0.5) refers to the mesh size of a single notional sieve allowing 50% of the total of all particles of the sample to pass. Thus d(0.5) = 30-210 µm means that the upper limit of the particle size range defining the notional half of the sample containing the smaller particles is between 30 µm to 210 µm. Thus 50% of the total of all particles have a particle size of not more than d(0.5) meaning in this case that they have a maximum size of 30 µm to 210 µm. Accordingly the parameter d(0.9) refers to the mesh size of a single notional sieve allowing 90% of the total of all particles of the sample to pass i.e. only 10% of the sample is retained. Thus d(0.9) = 70 - 400 µm means that the lower limit of the particle size range defining the 10% of largest particles in the sample is between 70 µm to 400 µm. Thus 90% of all particles have a particle size of not more than d(0.9) meaning in this case that they have a maximum size of 70 µm to 400 µm.

It is furthermore preferred that the particle size of the crystalline N-[5-(amino-sulfonyl)-4-methyl-1,3-thiazol-2-yl]-N-methyl-2-[4-(2-pyridinyl)-phenyl]acetamide mesylate monohydrate particles is within the range of 1 µm to 500 µm, preferably in the range of 1.5 µm to 450 µm and more preferably in the range of 2 µm to 400 µm. Thus, the particle size range (PSR) of the mesylate monohydrate is from 1.0 µm to 500 µm, preferably from 1.5 µm to 450 µm, more preferably from 2.0 µm to 400 µm, still more preferably from 2.5 µm to 300 µm and most preferably from 3.0 µm to 250 µm. If the PSR is not mentioned at all or if reference to the PSR is made without stating a definite value, it shall be referred to a particle size range from 1 to 500 µm.

Moreover it is preferred that the particle size distribution of the crystalline N-[5-(amino-sulfonyl)-4-methyl-1,3-thiazol-2-yl]-N-methyl-2-(4-(2-pyridinyl)-phenyl]acetamide mesylate monohydrate particles is characterized by d(0.1) from 4 to 100 µm, d(0.5) from 30 to 210 µm and d(0.9) from 70 to 400 µm, more preferably d(0.1) from 6 to 95 µm, d(0.5) from 50 to 200 µm and d(0.9) from 100 to 390 µm, still more preferably d(0.1) from 7 to 90 µm, d(0.5) from 70 to 190 µm and d(0.9) from 130 to 380 µm, still more preferably d(0.1) from 8 to 85 µm, d(0.5) from 80 to 185 µm and d(0.9) from 160 to 370 µm, still more preferably d(0.1) from 9 to 80 µm, d(0.5) from 90 to 180 µm and d(0.9) from 180 to 360 µm, still more preferably d(0.1) from 10 to 75 µm, d(0.5) from 100 to 175 µm and d(0.9) from 200 to 350 µm and most preferably d(0.1) from 11 to 70 µm, d(0.5) from 110 to 170 µm and d(0.9) from 220 to 340 µm.

Furthermore it is preferred that the specific surface area of the crystalline particles is less than 1.0 m²/g, more preferably less than 0.9 m²/g, still more preferably less than 0.8 m²/g, still more preferably less than 0.7 m²/g, still more preferably less than 0.6 m²/g, still more preferably less than 0.5 m²/g, still more preferably less than 0.4 m²/g and most preferably the SSA of the particles is less than 0.3 m²/g.

As used herein the term "mesylate monohydrate", or "crystalline mesylate monohydrate", or "methanesulfonic acid monohydrate", or "crystalline methanesulfonic acid monohydrate", or "N-[5-(amino-sulfonyl)-4-methyl-1,3-thiazol-2-yl]-N-methyl-2-[4-(2-pyridinyl)-phenyl]acetamide mesylate monohydrate", or "N-[5-(amino-sulfonyl)-4-methyl-1,3-thiazol-2-yl]-N-methyl-2-[4-(2-pyridinyl)-phenyl]acetamide methanesulfonic acid monohydrate" refers to the crystalline N-[5-(amino-sulfonyl)-4-methyl-1,3-thiazol-2-yl]-N-methyl-2-[4-(2-pyridinyl)-phenyl]-acetamide mono methanesulfonic acid monohydrate having the PSD, PSR and SSA as defined herein.

The crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to the invention is preferably combined with and used in combination with acetylsalicylic acid, trifluridine, idoxuridine, foscarnet, cidofovir, ganciclovir, aciclovir, penciclovir, valaciclovir, famciclovir and/or valganciclovir. Especially preferred are combinations with acetylsalicylic acid or aciclovir or penciclovir or acetylsalicylic acid and aciclovir or acetylsalicylic acid and penciclovir.

The inventive mesylate monohydrate or the afore-mentioned drug combinations are preferably used for the manufacture of a pharmaceutical composition for the treatment and/or prophylaxis of infectious diseases and the prevention of transmission of an infectious disease and especially infectious diseases caused by herpes simplex viruses.

The crystalline N-[5-(amino-sulfonyl)-4-methyl-1,3-thiazol-2-yl]-N-methyl-2-[4-(2-pyridinyl)-phenyl]acetamide mesylate monohydrate obtainable according to the above disclosed synthesis is then used to prepare a pharmaceutical composition, wherein the N-[5-(amino-sulfonyl)-4-methyl-1,3-thiazol-2-yl]-N-methyl-2-[4-(2-pyridinyl)phenyl]acetamide mesylate monohydrate has the particle size distribution (PSD), specific surface area (SSA) and particle size range (PSR) as defined herein. To this pharmaceutical composition acetylsalicylic acid, trifluridine, idoxuridine, foscarnet, cidofovir, ganciclovir, aciclovir, penciclovir, and/or their respective prodrugs valaciclovir, famciclovir and/or valganciclovir might be added. Some suppliers use the name acyclovir instead of aciclovir. Moreover, these pharmaceutical compositions are preferably solid pharmaceutical compositions without solvents, diluents, or liquids exhibiting solubilising properties for the active pharmaceutical ingredient so that the particles are not dissolved and remain unaltered in regard to their particle size distribution, particle size range and specific surface area.

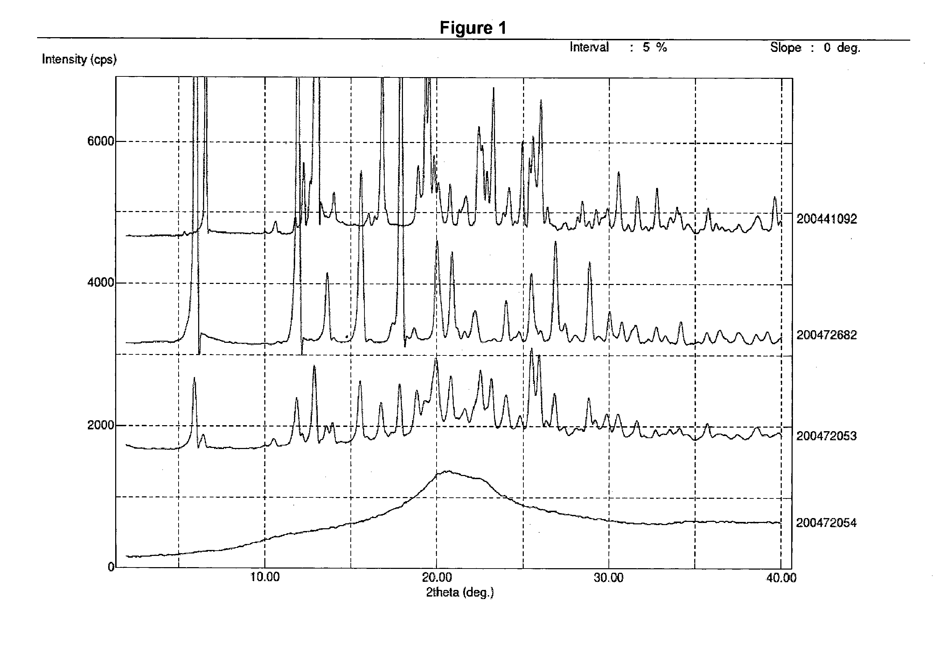

In WO2006/103011A a wet granulation process for tablet preparation is described for the base form using diverse acids including methanesulfonic acid. In a series of crystallization experiments starting from N-[5-(aminosulfonyl)-4-methyl-1,3-thiazol-2-yl]-N-methyl-2-[4-(2-pyridinyl)-phenyl]acetamide and benzoic acid, lactic acid, and sulphuric acid, a crystalline salt (apparently occurring in different polymorphic forms) could only be isolated with sulphuric acid. Nevertheless, using sulphuric acid in the wet granulation process resulted in a tablet exhibiting unfavourable dissolution properties (see Table 3 of WO2006/103011A). Repeating the wet granulation process (cf. example 5 of W02006/103011A) using 1 equivalent methanesulfonic as acidic component resulted in a granulate, which contained a mixture of crystalline free base and mesylate monohydrate salt (ca 90:10, detectable by comparison of the X-ray powder diffraction spectra, which did not change significantly over 4 weeks at 40°C (see Figure 1). This mesylate / free base mixture had been found unsuitable for a tablet formulation, because of low solubility and dissolution behaviour, the occurrence of at least four polymorphic forms and the additional risk of further interconversion between base and salt form under long term storage (also depending on the water content and hygroscopicity of the tablet mixture). Thus is was very surprising that a definite mesylate monohydrate salt of N-[5-(aminosulfonyl)-4-methyl-1,3-thiazol-2-yl]-N-methyl-2-[4-(2-pyridinyl)-phenyl]acetamide could be obtained and that this definite mesylate monohydrate salt of N-[5-(aminosulfonyl)-4-methyl-1,3-thiazol-2-yl]-N-methyl-2-[4-(2-pyridinyl)-phenyl]acetamide with the characteristic PSR, PSD and SSA as disclosed herein overcame all problems of the state of the art and provided the opportunity to prepare stable, long term stable, and pharmacologically applicable pharmaceutical compositions which showed clear advantages for solid formulations.

For the purpose of tabletting, the flowability of a mixture of N-[5-(aminosulfonyl)-4-methyl-1,3-thiazol-2-yl]-N-methyl-2-[4-(2-pyridinyl)-phenyl]acetamide mono methanesulfonic acid monohydrate having the PSR, PSD and SSA as defined herein with usual pharmaceutical ingredients is improved with larger particles and narrower particle size distribution. As particle size decreases, several interparticulate forces such as mechanical interlocking, hydrogen bonding, electrostatic, and van der Waals forces can predominate over gravity. These forces act in the surface of the particles and smaller particles have larger surface area in relation to their mass than larger ones. In addition, these varying results may be due to differences in the flowability measurement set-ups, humidity of the air, and/or particle properties such as original particle size. A broad particle size distribution induces segregation that influences tabletting force, tablet weigh and the content uniformity of the tablets. Broad particle size distribution causes more segregation problems during tabletting than a granule batch with smaller size distribution within a defined range. Thus due to various possible particle size ranges, particle size distributions and specific surface areas adjustable by various techniques, it was unforeseeable for the skilled person that the ranges for PSD, SSA and PSR as defined herein are the most suitable for preparing pharmaceutical compositions.

The narrow particle size distribution of the crystalline form of N-[5-(aminosulfonyl)-4-methyl-1,3-thiazol-2-yl]-N-methyl-2-[4-(2-pyridinyl)-phenyl]acetamide mono methanesulfonic acid monohydrate, e.g. for a given mean particle size, they have fewer large and small particles, is advantageous for direct compression as a method of tablet manufacturing. The resulting particle size distribution of the crystalline N-[5-(aminosulfonyl)-4-methyl-1,3-thiazol-2-yl]-N-methyl-2-[4-(2-pyridinyl)-phenyl]acetamide mono methanesulfonic acid monohydrate, wherein the PSR, PSD and SSA is as defined herein and preferably wherein the particle size distribution is defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, has physical characteristics, which are particularly adapted to be able to allow manufacturing by direct compression without a wet or dry granulation step.

According to the invention, it is preferred that at least 65 v/v%, more preferably at least 70 v/v%, even more preferably at least 80 v/v%, even more preferably at least 85 v/v%, even more preferably at least 90 v/v%, even more preferably at least 95 v/v% and most preferred at least 99 v/v% of crystalline N-[5-(aminosulfonyl)-4-methyl-1,3-thiazol-2-yl]-N-methyl-2-[4-(2-pyridinyl)-phenyl]acetamide mono methanesulfonic acid monohydrate particles fall in a particle size range of from 2 to 400 µm. Moreover it is preferred that 40 v/v% N-[5-(aminosulfonyl)-4-methyl-1,3-thiazol-2-yl]-N-methyl-2-[4-(2-pyridinyl)-phenyl]acetamide methane-sulfonic acid monohydrate particles fall in a particle size range of from 2 - 250 µm.

It was surprisingly found that by using crystalline form of N-[5-(aminosulfonyl)-4-methyl-1,3-thiazol-2-yl]-N-methyl-2-[4-(2-pyridinyl)-phenyl]acetamide mono methanesulfonic acid monohydrate in particulate form, wherein the PSR, PSD and SSA is as defined herein and preferably wherein the particle size distribution is defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g in the final blend shows improved free-flowing and cohesive powder characteristics and displays cohesive briquetting and dry granulation behaviour, which effects are significant for efficient and robust direct compression. If too many crystalline N-[5-(aminosulfonyl)-4-methyl-1,3-thiazol-2-yl]-N-methyl-2-[4-(2-pyridinyl)-phenyl]acetamide mono methanesulfonic acid monohydrate particles are present whose particle size is lower than about 4 µm, picking and sticking tend to be caused in the later processing. If too many crystalline N-[5-(aminosulfonyl)-4-methyl-1,3-thiazol-2-yl]-N-methyl-2-[4-(2-pyridinyl)-phenyl]acetamide mono methanesulfonic acid monohydrate particles are present whose particle size is larger than about 400 µm, the compressibility becomes too poor. Therefore, it is observed that at least 65 v%/v% of crystalline N-[5-(aminosulfonyl)-4-methyl-1,3-thiazol-2-yl]-N-methyl-2-[4-(2-pyridinyl)-phenyl]acetamide mono methanesulfonic acid monohydrate fall in a particle size range of from 2 to 400 µm, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g. When processing crystalline N-[5-(aminosulfonyl)-4-methyl-1,3-thiazol-2-yl]-N-methyl-2-[4-(2-pyridinyl)-phenyl]acetamide mono methanesulfonic acid monohydrate, its particle size distribution remains substantially unchanged, typically it remains totally unchanged. In view of improved overall characteristics, it is preferred that at least 65 v/v%, more preferably at least 70 v/v%, even more preferably at least 80 v/v%, even more preferably at least 85 v/v%, even more preferably at least 90 v/v%, even more preferably at least 95 v/v% and most preferred at least 99 v/v% of crystalline N-[5-(aminosulfonyl)-4-methyl-1,3-thiazol-2-yl]-N-methyl-2-[4-(2-pyridinyl)-phenyl]acetamide mono methanesulfonic acid monohydrate particles have the PSR, PSD and SSA as defined herein and more preferably fall in a particle size range of from 2 to 400 µm, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and still more preferably defined by d(0.1) from 10 to 75 µm, d(0.5) from 100 to 175 µm, d(0.9) from 200 to 350 µm with a specific surface area of the particles less than 0.3 m²/g.

A further advantage of the tablets according to the invention is that the tablet will have an optimised dissolution rate based on its particle size distribution of the crystalline form of N-[5-(aminosulfonyl)-4-methyl-1,3-thiazol-2-yl]-N-methyl-2-[4-(2-pyridinyl)-phenyl]acetamide mono methanesulfonic acid monohydrate which has the PSD, PSR and SSA as defined herein and thus, the drug may be absorbed into the blood stream much faster compared to N-[5-(aminosulfonyl)-4-methyl-1,3-thiazol-2-yl]-N-methyl-2-[4-(2-pyridinyl)-phenyl]acetamide or salts thereof. Furthermore, the surprising dispersion times obtained with tablets according to the invention are advantageous for swallowable tablets. In a further embodiment, the tablets according to the invention can be presented for dispersion in water.

The crystalline mesylate monohydrate salt of N-[5-(aminosulfonyl)-4-methyl-1,3-thiazol-2-yl]-N-methyl-2-[4-(2-pyridinyl)phenyl]acetamide, wherein the PSR, PSD and SSA is as defined herein, exhibits increased long term stability properties and a desired release kinetic and long term stability from pharmaceutical compositions, which is superior to other salts of N-[5-(aminosulfonyl)-4-methyl-1,3-thiazol-2-yl]-N-methyl-2-[4-(2-pyridinyl)phenyl]acetamide, which are known in the state of the art including also other mesylate salts.

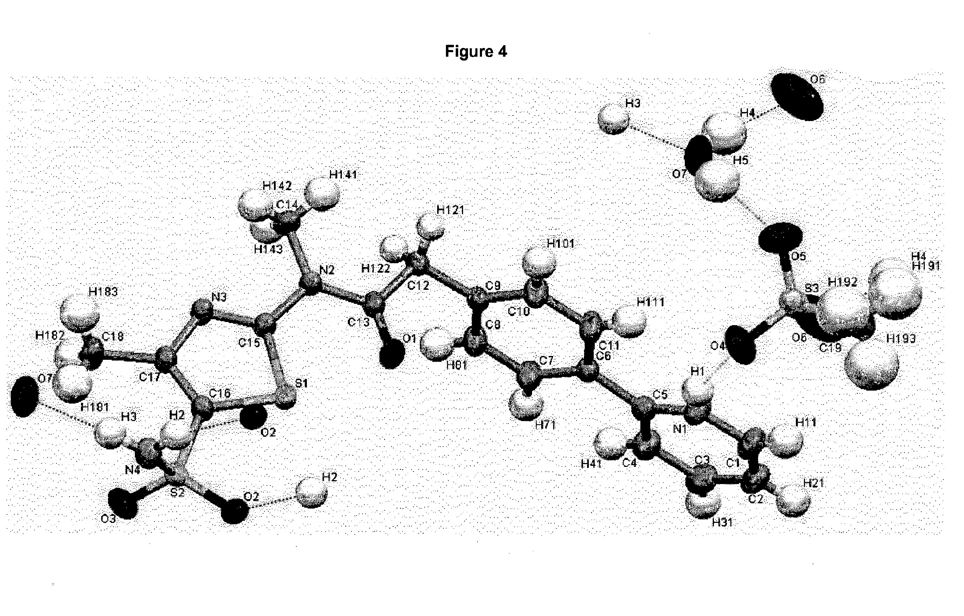

As evident from Figure 4 that shows the single-crystal X-ray structure analysis of the N-[5-(aminosulfonyl)-4-methyl-1,3-thiazol-2-yl]-N-methyl-2-[4-(2-pyridinyl)-phenyl]-acetamide mono methanesulfonic acid monohydrate, the salt is formed between the mesylate and the protonated pyridinyl ring. Moreover, exactly one mol equivalent methanesulfonic acid and one mol equivalent water is incorporated into the crystal structure, wherein the hydrogen atoms of the water molecule form hydrogen bridges with oxygen atoms of two different mesylate molecules.

Preferred conditions for the crystallization of N-[5-(aminosulfonyl)-4-methyl-1,3-thiazol-2-yl]-N-methyl-2-[4-(2-pyridinyl)-phenyl]acetamide mono methanesulfonic acid monohydrate are preparing a suspension of the base in about 10 vol. (vol. = L/kg of free base) ethanol / water (1:1), adding 1.15 equivalents of methanesulfonic acid at 50 - 55°C during < 15 min, seeding with 0.5 mol % of final product, ageing for 1-1.5 h at 50°C and cooling to 20 - 25°C during 2.5 h.

After further stirring for 1 h, the final product can be isolated by filtration and drying *in vacuo,* resulting in a yield of > 95 %. Using this procedure, N-[5-(amino-sulfonyl)-4-methyl-1,3-thiazol-2-yl]-N-methyl-2-[4-(2-pyridinyl)-phenyl]acetamide methane-sulfonic acid monohydrate having the PSR, PSD and SSA is as defined and preferably having a particle size range of from 2 to 400 µm, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g in purity >99% containing < 2 ppm residual Pd could be prepared reproducibly concerning yield, purity, polymorphic form, PSD, PSR and SSA from a supersaturated solution of N-[5-(aminosulfonyl)-4-methyl-1,3-thiazol-2-yl]-N-methyl-2-[4-(2-pyridinyl)-phenyl]acetamide by crystallization under controlled conditions as described below.

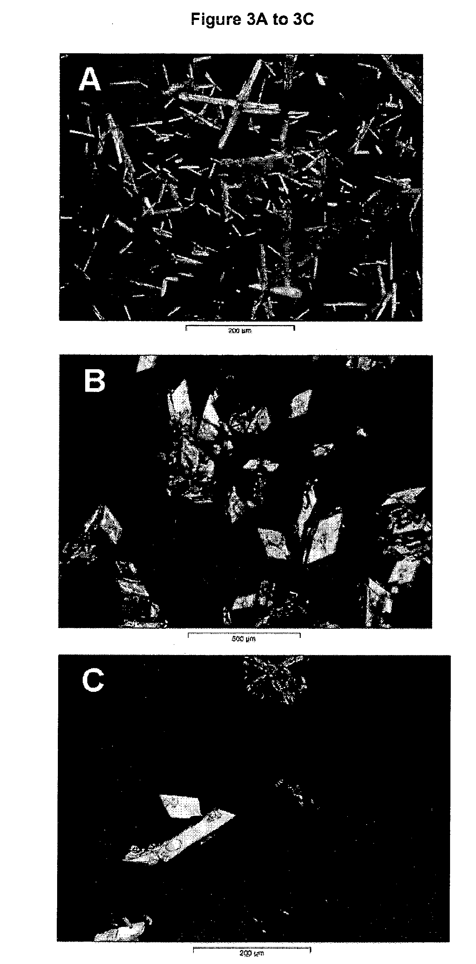

Spontaneous crystallization from an over-saturated solution results in a coprecipitation of free base. To simulate this process, a sample of N-[5-(amino-sulfonyl)-4-methyl-1,3-thiazol-2-yl]-N-methyl-2-[4-(2-pyridinyl)phenyl] acetamide mono methanesulfonic acid monohydrate was dissolved in a mixture of ethanol and water (1:1) at 50°C an then cooled down and stirred at 0°C for ca 5 h. Samples of the precipitate were taken for microscopy exhibiting mainly small needles (Figure 3A) in contrast to the mesylate monohydrate, which crystallizes as prisms (Figure 3B). By investigating isolated needles applying ¹H NMR (nuclear magnetic resonance) no mesylate could be detected, thus exhibiting the existence of mainly free base form. Keeping a suspension of this precipitate at 0°C or room temperature shows slow growth of the prisms consuming the needles (Figure 3C), which argues for a very slow conversion to the mesylate salt form. This experiment demonstrates the importance of selection of well-defined crystallization conditions.

To obtain N-[5-(amino-sulfonyl)-4-methyl-1,3-thiazol-2-yl]-N-methyl-2-[4-(2-pyridinyl)phenyl] acetamide mono methanesulfonic acid monohydrate in the desired PSD, PSR and SSA, also moderate to slow stirring of this mixture and a flat cooling ramp of this mixture to room temperature are preferred. Furthermore it is preferred to add the methanesulfonic acid over 5 - 15 minutes at elevated temperature and to keep the resulting mixture at this elevated temperature for 1 to 2 hours after completion of the addition of methanesulfonic acid. The cooling to room temperature is performed within 2 to 3 hours and the mixture is thereafter slowly stirred for another hour at room temperature. Then the crystals are filtered off, washed with alcohol/water and dried under vacuum at a temperature between 20°C and 60°C, preferably starting at 20°C and ending at 60°C.

A modified way to obtain N-[5-(amino-sulfonyl)-4-methyl-1,3-thiazol-2-yl]-N-methyl-2-[4-(2-pyridinyl)phenyl] acetamide mono methanesulfonic acid monohydrate in the desired PSD, PSR and SSA applies the following conditions for the crystallization. Methanesulfonic acid is added at elevated temperatures, and preferably between 30°C and 90°C, more preferably between 35°C and 80°C, still more preferably between 40°C and 70°C, still more preferably between 45°C and 60°C and most preferably at 50°C - 55°C to the mixture of an organic solvent and water containing N-[5-(aminosulfonyl)-4-methyl-1,3-thiazol-2-yl]-N-methyl-2-[4-(2-pyridinyl)phenyl]-acetamide yielding a supersaturated solution of the mesylate of N-[5-(aminosulfonyl)-4-methyl-1,3-thiazol-2-yl]-N-methyl-2-[4-(2-pyridinyl)phenyl]-acetamide. Organic solvents which are miscible or consolute with water are preferred such as MeOH, EtOH, n-PrOH, I-PrOH, acetonitrile, THF, acetone. Moreover it is preferred to add seed crystals of N-[5-(aminosulfonyl)-4-methyl-1,3-thiazol-2-yl]-N-methyl-2-[4-(2-pyridinyl)phenyl]acetamide mono methane-sulfonic acid monohydrate to this supersaturated mixture also at elevated temperatures like 30°C to 90°C, preferably 35°C to 80°C, more preferably 40°C to 70°C, still more preferably 45°C to 60°C and most preferably at 50°C - 55°C. Also moderate to slow stirring of this mixture and a slow cooling of this mixture to room temperature are preferred. Furthermore it is preferred to add the methanesulfonic acid over 5 to 15 minutes at the elevated temperature and to keep the resulting mixture at this elevated temperature for 0.5 to 5 hour and more preferably 1 to 2 hours after completion of the addition of the methanesulfonic acid. The cooling to room temperature is performed within 1 to 5 hour and preferably 2 to 3 hours and the mixture is thereafter slowly stirred for preferably another hour at room temperature. Then the crystals are filtered off, washed with alcohol/water and preferably dried under vacuum at a temperature between 20°C and 60°C, preferably starting at 20°C and ending at 60°C

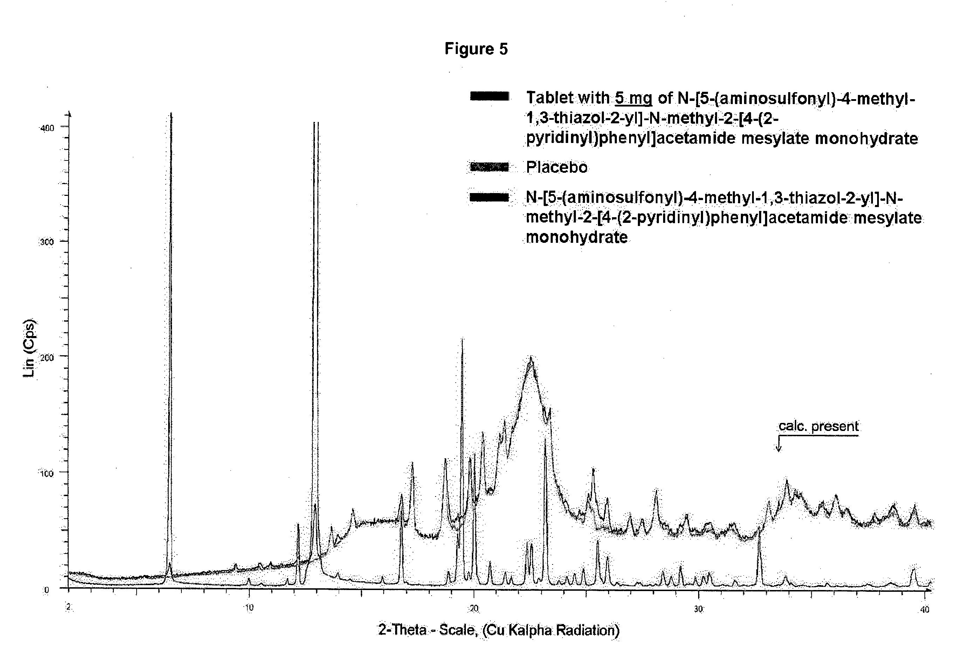

The crystalline N-[5-(aminosulfonyl)-4-methyl-1,3-thiazol-2-yl]-N-methyl-2-[4-(2-pyridinyl)-phenyl]acetamide mono methanesulfonic acid monohydrate with the PSD, PSR and SSA as defined herein, exhibits optimised release kinetics from pharmaceutical compositions and improved bioavailability. Furthermore, storage properties, especially the long-term stability of the active pharmaceutical ingredient (API) as well as of the pharmaceutical formulations are excellent. X-ray powder diffraction spectra of film coated tablets (strengths 5 mg, 25 mg, 100 mg, powdered for measurement in comparison to placebo) after 24 months at room temperature revealed no change in crystallinity (see Figures 5 to 7).

Compared to the mesylate salt of N-[5-(aminosulfonyl)-4-methyl-1,3-thiazol-2-yl]-N-methyl-2-[4-(2-pyridinyl)phenyl]acetamide, the monohydrate of the mesylate salt of N-[5-(aminosulfonyl)-4-methyl-1,3-thiazol-2-yl]-N-methyl-2-[4-(2-pyridinyl)-phenyl]acetamide, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 1.0 m²/g, preferably less than 0.6 m²/g, and most preferably less than 0.3 m²/g, surprisingly and unexpectedly has a greater thermostability of up to 170°C. A clear advantage of the N-[5-(aminosulfonyl)-4-methyl-1,3-thiazol-2-yl]-N-methyl-2-[4-(2-pyridinyl)-phenyl]acetamide mesylate monohydrate, wherein the PSD, PSR and SSA are as defined herein, is its proven long-term stability. Therefore, the inventive N-[5-(aminosulfonyl)-4-methyl-1,3-thiazol-2-yl]-N-methyl-2-[4-(2-pyridinyl)phenyl]acetamide mesylate monohydrate, which is characterized by the PSD, PSR and SSA as disclosed herein, is better suited for the preparation of long-term stable tablets compared to the free base N-[5-(aminosulfonyl)-4-methyl-1,3-thiazol-2-yl]-N-methyl-2-[4-(2-pyridinyl)phenyl]acetamide.

The crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to the invention is a useful compound for treatment and/or prophylaxis of infectious diseases and/or prevention of transmission of infectious diseases.

The crystalline N-[5-(aminosulfonyl)-4-methyl-1,3-thiazol-2-yl]-N-methyl-2-[4-(2-pyridinyl)-phenyl]acetamide mono methanesulfonic acid monohydrate is highly active against herpesviruses and infections caused by herpesviruses and/or transmission of a herpes virus or herpes viruses. Therefore, the inventive crystalline N-[5-(aminosulfonyl)-4-methyl-1,3-thiazol-2-yl]-N-methyl-2-[4-(2-pyridinyl)-phenyl]acetamide mono methanesulfonic acid monohydrate is especially useful for the preparation of a pharmaceutical composition for the treatment and/or prophylaxis of diseases, which are caused by herpesviruses or caused by the transmission of a herpes virus or herpes viruses.

The inventive mesylate monohydrate salt is especially useful for the treatment and/or prophylaxis of infections, which are caused by herpes simplex viruses, or for the prevention of transmission of a herpes virus or herpes viruses. Infections with herpes simplex viruses (HSV, subtype 1 and 2) are categorized into one of several distinct disorders based on the site of infection. Orofacial herpes simplex infection, the visible symptoms of which are colloquially called cold sores or fever blisters, affects the face and mouth. Orofacial herpes is the most common form of infection. Genital herpes is the second common form of a herpes simplex infection. Although genital herpes is largely believed to be caused by HSV-2 only, genital HSV-1 infections are increasing. Other disorders such as herpetic whitlow, herpes gladiatorum, ocular herpes (keratitis), cerebral herpes infection encephalitis, Mollaret's meningitis, neonatal herpes, and possibly Bell's palsy are also caused by herpes simplex viruses.

The inventive mesylate monohydrate salt is thus useful for the treatment and/or prophylaxis of infections which are caused by herpes simplex viruses and/or for the prevention of transmission of herpes simplex viruses.

The mesylate monohydrate salt of the present invention can be combined and administered together with an anti-inflammatory agent such as acetylsalicylic acid and acetaminophen. Thus combinations of the inventive mesylate monohydrate with acetylsalicylic acid and/or acetaminophen as well as pharmaceutical compositions containing such a combination are preferred.

Furthermore, the inventive mesylate monohydrate can be combined and can be used in combination with an anti-viral agent. The anti-viral agent is preferably an antimetabolite and most preferably a nucleobase analogue, nucleotide analogue or nucleoside analogue drug. It is further preferred if the anti-viral agent is useful against herpesviruses and/or against transmission of a herpes virus or herpes viruses and is selected from the group of drugs comprising but not limited to or consisting of: trifluridine, idoxuridine, foscarnet, cidofovir, ganciclovir, aciclovir or penciclovir or the respective prodrugs valaciclovir, famciclovir or valganciclovir.

The combination of the inventive mesylate monohydrate and a further active agent like an anti-inflammatory, immunomodulatory, or anti-viral agent, such as therapeutic vaccines, siRNAs, antisense oligonucleotides, nanoparticles or virus- uptake inhibitors such as n-docosanol, may be administered simultaneously in one single pharmaceutical composition or in more than one pharmaceutical composition, wherein each composition comprises at least one active agent.

The pharmaceutical compositions of the present invention can be prepared in a conventional solid and a conventional pharmaceutically-made adjuvant at suitable dosage level in a known way. Preferred preparations may be adapted for oral application. These administration forms include, for example, pills, tablets, film tablets, coated tablets, sustained release formulations, and capsules.

The pharmaceutical compositions according to the invention preferably comprise 5 to 70% by weight more preferably 10 to 30% by weight crystalline N-[5-(aminosulfonyl)-4-methyl-1,3-thiazol-2-yl]-N-methyl-2-[4-(2-pyridinyl)-phenyl]acetamide mono methanesulfonic acid monohydrate (all percentage data are percentages by weight based on the weight of the pharmaceutical preparation), wherein the PSD, PSR and SSA is as defined herein. The pharmaceutical composition comprises usually 2 to 600 mg of crystalline N-[5-(aminosulfonyl)-4-methyl-1,3-thiazol-2-yl]-N-methyl-2-[4-(2-pyridinyl)-phenyl]acetamide mono methanesulfonic acid monohydrate, preferably 5 to 500 mg, more preferably 10 to 300 mg and particularly preferably 20 to 200 mg, wherein PSD, PSR and SSA is as disclosed above.

The pharmaceutical composition according to the invention optionally comprises one or more filler, which, for example, are selected from the group consisting of: Microcrystalline cellulose, fiber cellulose, calcium phosphates and mannitol. Preferably, according to the invention, microcrystalline cellulose and mannitol are used. The pharmaceutical composition expediently comprises 20 to 80%, preferably 40 to 80%, particularly preferably 45 to 70% microcrystalline cellulose and 1 to 40%, preferably 5 to 30%, particularly preferably 10 to 20% mannitol. The pharmaceutical preparation according to the invention may comprise at least one disintegration auxiliary, which is, for example, selected from the group consisting of starch, pre-gelatinized starch, starch glycolates, cross-linked polyvinylpyrrolidone, sodium carboxymethylcellulose (= croscarmellose sodium) and other salts of carboxymethylcellulose. A mixture of two disintegration agents can also be used. According to the invention, the use of croscarmellose sodium is preferred. The pharmaceutical composition expediently comprises 3 to 35%, preferably 5 to 30% and particularly preferably 5 to 10% of the disintegration auxiliary(ies). The pharmaceutical composition of the invention may comprise at least one lubricant selected from the group consisting of fatty acids and their salts. According to the invention, the use of magnesium stearate is particularly preferred.

The pharmaceutical composition of the invention may comprise a flow agent, which could be colloidas anhydrous silica or talcum powder. According to the invention, the use of colloidas anhydrous silica is particularly preferred. The flow agent is expediently used in an amount of 0.3 to 2.0%, particularly preferably from 0.4 to 1.5% and most preferably from 0.5 to 1 %.

A particularly preferred pharmaceutical composition of the invention comprises: 5% - 30% crystalline N-[5-(aminosulfonyl)-4-methyl-1,3-thiazol-2-yl]-N-methyl-2-[4-(2-pyridinyl)phenyl]acetamide mono methanesulfonic acid monohydrate, wherein the PSD, PSR and SSA is as defined herein and preferably the particle size distribution is defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, 5% - 10% croscarmellose-sodium, 0.5 to 0.7% magnesium stearate, 40% - 70% microcrystalline cellulose, 10% - 20% mannitol and 0.5% to 1% colloidal anhydrous silica.

Further pharmaceutical compositions according to the invention preferably comprise 30 to 90% more preferably 50 to 70% by weight crystalline N-[5-(aminosulfonyl)-4-methyl-1,3-thiazol-2-yl]-N-methyl-2-[4-(2-pyridinyl)-phenyl]acetamide mono methanesulfonic acid monohydrate, wherein the PSD, PSR and SSA is as defined herein and preferably the particle size distribution is defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g (all percentage data are percentages by weight based on the weight of the pharmaceutical preparations). The pharmaceutical composition comprises usually 20 to 750 mg of crystalline N-[5-(aminosulfonyl)-4-methyl-1,3-thiazol-2-yl]-N-methyl-2-[4-(2-pyridinyl)-phenyl]acetamide mono methanesulfonic acid monohydrate, wherein the PSD, PSR and SSA is as defined herein and preferably 50 to 500 mg and particularly preferably 50 to 250 mg based on a single dosage.

The pharmaceutical composition according to the invention optionally comprises one or more dry binders, which, for example, are selected from the group consisting of microcrystalline cellulose, fiber cellulose, calcium phosphates, and mannitol. Preferably, microcrystalline cellulose is used. This is commercially available under the designation AvicelB, for example. The pharmaceutical composition expediently comprises 1 to 20%, preferably 1 to 10%, particularly preferably 1 to 5% of the dry binder(s). The pharmaceutical preparation according to the invention may comprise at least one disintegration auxiliary which is for example selected from the group consisting of starch, pre-gelatinized starch, starch glycolates, cross-linked polyvinylpyrrolidone, sodium carboxymethylcellulose (= croscarmellose sodium) and other salts of carboxymethylcellulose. A mixture of two disintegration agents can also be used. The use of croscarmellose sodium and cross-linked polyvinylpyrrolidone or a mixture of the two is preferred. The pharmaceutical composition expediently comprises 3 to 35%, preferably 10 to 30% and particularly preferably 15 to 25% of the disintegration auxiliary(ies). The pharmaceutical preparation of the invention may comprise at least one lubricant selected from the group consisting of fatty acids and their salts. The use of magnesium stearate is particularly preferred.

A pharmaceutical composition for a tablet with 25 mg of the active pharmacologic ingredient (calculated as base form) comprises 32.3 mg of N-[5-(aminosulfonyl)-4-methyl-1,3-thiazol-2-yl]-N-methyl-2-[4-(2-pyridinyl)-phenyl]acetamide mono methanesulfonic acid monohydrate, wherein the PSD, PSR and SSA is as defined herein and preferably the particle size distribution is defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, 60.9 mg of microcrystalline cellulose, 9.8 mg of croscarmellose sodium, 20.0 mg of mannitol, 1.3 mg of colloidal anhydrous silica, and 0.9 mg of magnesium stearate. The total weight of the blend is 125.0 mg.

A further pharmaceutical composition for a tablet with 100 mg of the active pharmacologic ingredient comprises 129.0 mg of the inventive N-[5-(aminosulfonyl)-4-methyl-1,3-thiazol-2-yl]-N-methyl-2-[4-(2-pyridinyl)-phenyl]acetamide mono methanesulfonic acid monohydrate, 243.4 mg of microcrystalline cellulose, 39.0 mg of croscarmellose sodium, 80.1 mg of mannitol, 5.0 mg of colloidal anhydrous silica, and 3.5 mg of magnesium stearate. The total weight of the blend is 500.0 mg.

Furthermore, the present invention also includes pharmaceutical compositions for sublingual application, which preparations in addition to typical vehicles contain at least crystalline N-[5-(aminosulfonyl)-4-methyl-1,3-thiazol-2-yl]-N-methyl-2-[4-(2-pyridinyl)-phenyl]acetamide mono methanesulfonic acid monohydrate, wherein the PSD, PSR and SSA is as defined herein, as active ingredient.

An inventive pharmaceutical composition may contain the following preservatives: phenoxyethanol, formaldehyde solution, parabens, pentanediol, or sorbic acid.

As pharmaceutically acceptable carrier, excipient and/or diluents can be used carriers such as preferably an inert carrier like lactose, starch, sucrose, cellulose, magnesium stearate, dicalcium phosphate, calcium sulfate, talc, mannitol, ethyl alcohol (liquid filled capsules); suitable binders include starch, gelatin, natural sugars, corn sweeteners, natural and synthetic gums such as acacia, sodium alginate, carboxymethylcellulose, polyethylene glycol and waxes, sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate; lubricants such as boric acid, sodium benzoate, sodium acetate, sodium chloride, magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine; disintegrating agents (disintegrates) such as starch, methylcellulose, guar gum, modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures; coloring agents, sweetening agents, flavoring agents, preservatives; glidents are for example silicon dioxide and talc; suitable adsorbent are clay, aluminum oxide, suitable diluents are water or water/propylene glycol solutions for parenteral injections, juice, sugars such as lactose, sucrose, mannitol, and sorbitol, starches derived from wheat, corn rice, and potato, and celluloses such as microcrystalline cellulose.

The following examples are included to demonstrate preferred embodiments of the invention. It should be appreciated by those of skill in the art that the techniques disclosed in the examples, which follow, represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the present invention.

The pharmaceutical compositions according to the invention can be administered to a patient in need thereof at a once daily dose of about 20 to 750 mg of crystalline N-[5-(aminosulfonyl)-4-methyl-1,3-thiazol-2-yl]-N-methyl-2-[4-(2-pyridinyl)-phenyl]acetamide mono methanesulfonic acid monohydrate, wherein the PSD, PSR and SSA is as defined herein and preferably the particle size distribution is defined by d(0.1) from 2 to 100 µm, d(0.5) from 30 to 210 µm and d(0.9) from 70 to 400 µm with a specific surface area of the particles less than 1.0 m²/g , and more preferably defined by d(0.1) from 10 to 75 µm, d(0.5) from 100 to 175 µm, d(0.9) from 200 to 350 µm with a specific surface area of the particles less than 0.3 m²/g, preferably 50 to 500 mg and particularly preferably 50 to 250 mg based on a single dosage. The pharmaceutical compositions according to the invention can also be administered to a patient in need thereof thrice daily, twice daily, once daily, thrice weekly, twice weekly, or once weekly. The administration on a thrice weekly, twice weekly, or once weekly basis is preferred and especially preferred is a once weekly administration, i.e. an administration one time a week of a pharmaceutical composition containing between 400 mg to 500 mg of the inventive N-[5-(aminosulfonyl)-4-methyl-1,3-thiazol-2-yl]-N-methyl-2-[4-(2-pyridinyl)-phenyl]acet-amide mono methanesulfonic acid monohydrate. Moreover it is preferred to start the administration of the mesylate monohydrate of the present invention with a high loading dose, for instance, with an initial single dose of 400 mg to 800 mg and to continue the administration with a lower dose of 100 mg to 150 mg per day or per week over the period of treatment.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the present invention as described in the following claims.

### EXAMPLES

### Example 1: Synthesis of N-[5-(aminosulfonyl)-4-methyl-1,3-thiazol-2-yl]-N-methyl-2-[4-(2-pyridinyl)-phenyl]acetamide methanesulfonate monohydrate having the inventive PSD, PSR and SSA

### Step 1 (Suzuki-Miyaura coupling and saponification)

The inertized reactor is charged with bis(triphenylphosphine)palladium(II) chloride (0.010 eq.) and reinertized. Then, toluene (1.65 vol.) is added. After heating to 40°C, triethylamine (3.00 eq.) is added. A solution of ethyl-4-bromophenylacetate (1.00 eq.) in toluene (0.82 vol.) is added. The resulting suspension is heated to 90-95°C prior to dosing pinacol borane (1.30 eq.) over a period of 60-90 min. Stirring at 90-95°C is continued for at least 2 more h before conversion is checked by HPLC. After cooling to 10°C, 2-chloropyridine (1.00 eq.) is charged to the reaction mixture. Then, 30% NaOH (6.00 eq.) is added followed by heating to 55-60°C. Stirring at this temperature is continued for at least 4 h before conversion is checked by HPLC. Once conversion is deemed complete, the reaction mixture is concentrated at about 300 mbar until 0.8 vol. (vol. refers to kg of starting material (= 1.00 eq) in the respective step, i.e. L/kg starting material) of distillate have been collected. The reaction mixture is diluted with water (2.72 vol.), cooled to 20°C and the phases are separated. The organic layer is discarded, while the pH of the aqueous layer is adjusted to pH 1 by addition of 33% HCl at 20°C. MIBK (2.30 vol.) and Celite (165 g/kg) are added and the resulting mixture is stirred for at least 15 min at 20°C before the solids are removed by filtration. The reactor and the filter cake are rinsed successively with water and the combined filtrate is transferred back into the reactor. The phases are separated and the aqueous layer is washed twice more with MIBK. After dilution with water, the aqueous acidic product solution was heated to 55°C and filtered through a plug packed with Celite at the bottom and activated charcoal on top. The Celite/charcoal plug was washed once more with pre-heated water (0.5 vol., 55°C) and the combined filtrate was charged back into the reactor. At 20°C, the pH was adjusted to ∼3.0 by addition of 30% NaOH before the product solution was heated to 60°C. More NaOH was dosed to adjust the pH to 4.1-4.3. The resulting suspension was stirred for 1-1.5 h at 60°C prior to being cooled to 20°C. After additional stirring for at least 1 h at this temperature, the product was filtered, washed twice with water, pre-dried in a flow of N₂ and finally dried in vacuum at 50-65°C. Typical yield: 38-41 %.

### Step 2 (amide coupling)

The reactor is charged with product from step 1 (1.00 eq.) and 4-methyl-2-(methylamino)-1,3-thiazole-5-sulfonamide (1.02 eq.). THF (7.08 vol.) and NMP (1.11 vol.) are added. The resulting suspension is cooled to 0°C prior to adding 1-ethyl-3-(3-dimethyllaminopropyl)carbodiimide hydrochloride (1.23 eq.) in 4 equal portions over a period of > 90 min. After at least 2 more h at 0°C, the reaction mixture is warmed to 20°C. At this temperature, stirring is continued for additional 2 h before conversion is checked by HPLC. Then, at 10-15°C about 2% (0.2 vol.) of the reaction mixture are added to water (12.3 vol.) within at least 5 min. The resulting thin suspension is stirred at 10-15°C for at least 1 h prior to dosage of the remaining bulk of the reaction mixture over > 4 h. Stirring at 10-15°C is continued for at least 0.5 h before the solids are filtered off, washed with water and dried on a nutsche filter in a steady flow of N₂ until deemed sufficiently dry (LOD < 45 % w/w; LOD: Loss on drying).

The feed reactor is charged with the crude product, THF (8.15 vol.), and water (up to 1.17 vol. depending on the loss on drying of crude product). The resulting suspension is heated to 60-65 °C and stirred for 1 h at this temperature. An almost clear solution is obtained which is subjected to polish filtration using a heatable lense filter heated to 60°C. The feed reactor, the transfer lines and the filter are successively rinsed with a mixture of THF (0.44 vol.) and purified water (0.06 vol.) at 60-65°C. The combined filtrate is collected in a separate reactor and heated to 50-55°C. To the reactor content, water (3.23 vol.) is dosed over at least 30 min. Stirring at 50-55°C is continued for 1-1.5 h before another portion of water (8.93 vol.) is slowly added within 2 h. After stirring for 1-1.5 h at 50°C, the resulting suspension is cooled to 5°C over 2.5 h and stirred for further 0.5 h. Then, the solids are filtered off, washed with water (3 x 2.96 vol.) and pre-dried on the nutsche filter in a steady flow of N₂. Final drying is accomplished *in vacuo* at 50-65°C using a conical drier. Typical yield: 78-83%.

### Step 3 (Salt Formation)

The reactor is charged with product from step 2 (1.00 eq.), ethanol (4.96 vol.) and water (4.96 vol.). After heating the resulting suspension to 50-55°C, methanesulfonic acid (1.15 eq.) is added within < 15 min. Complete dissolution of starting materials is typically observed at the very end of addition. Immediately within the next 5 min, stirring is reduced to the minimum acceptable rate and the reaction mixture is seeded with N-[5-(aminosulfonyl)-4-methyl-1,3-thiazol-2-yl]-N-methyl-2-[4-(2-pyridinyl)-phenyl]acetamide methanesulfonate monohydrate (0.005 eq.) which was prepared in the desired polymorphic form in a preceding experiment. Slow stirring at 50-55°C is continued for 60 to 90 min prior to cooling down to 20 to 25°C during > 2.5 h. After stirring for 1 more h, the solids are filtered off, washed with ethanol/water 5:2 V/V (3.10 vol.), pre-dried in a nitrogen flow and transferred into a conical drier for final drying *in vacuo* at 20 to 60°C.
Typical yield: >95%.

### Particle Analysis and Detection

A Malvern Mastersizer 2000 with a dispersion unit Malvern Hydro 2000S was used for liquid dispersion. Dispersing agent was Span™ 80 used at a concentration of 0.1% (volume/volume) in n-heptane. Stirring rate was 2,500 rounds per minute. Typically, a slurry of 50 milligrams of N-[5-(aminosulfonyl)-4-methyl-1,3-thiazol-2-yl]-N-methyl-2-[4-(2-pyridinyl)phenyl]acetamide mono methanesulfonic acid monohydrate was prepared in a vial containing 4 to 5 mL dispersant. Should any agglomerates have occurred, then these were converted to primary particles by ultrasonic treatment for 10 seconds. Those with ordinary skills in the art may recognise that ultrasonic treatment is common practice in converting agglomerates to smaller particles. Once the suspension had been generated, the suspension was transferred to the Malvern Mastersizer 2000 with a dispersion unit Hydro 2000S by pipetting, until an optimal concentration range had been reached. The optimal concentration range was assessed by the obscuration value displayed by the Malvern Mastersizer 2000. Typically, optimal obscuration values are in the range from about 10 to about 20 percent. Then, the measurement was performed with automatic subtraction of the background. Data were analysed automatically using the Fraunhofer diffraction equation. For microscopy, either the slurry used for particle size analysis or a new batch was used. The size of the observed particles was determined by means of the software *analySIS start 5.0* (Olympus Soft Imaging Solutions GmbH). The procedure described above complies with USP 429 (light diffraction measurement of particle size) and Ph. Eur. 2.9.31 (particle size analysis by laser light diffraction). In addition to particle size analysis by laser light diffraction, an optical investigation using a microscope was conducted. In case of release measurements, system suitability testing must be performed prior to dispersing, which is common practice for those skilled in the art.

### Example 2:

The trials disclosed by example 2 were conducted in order to investigate the influence of the particle size distribution (PSD) of the active pharmaceutical ingredient (API) on the dissolution properties of the tablet cores.

The term "particle size distribution" of a powder, or granular material, or particles dispersed in fluid, as used within this application, is a list of values or a mathematical function that defines the relative amounts of particles present, sorted according to size. The d(0.1), d(0.5) and d(0.9) values indicate that 10%, 50% and 90% of the particles measured were less than or equal to the size stated. For example, values of d(0.1)=52 , d(0.5)=129 and d(0.9)=257 mean that 10% of the particles were less than or equal to 52 µm, 50% were less than or equal to 129 µm and 90% were less than or equal to 257 µm (cf. Table 1, trial batch number G05). In order to study the dissolution properties of the tablet cores with different PSD of the inventive N-[5-(aminosulfonyl)-4-methyl-1,3-thiazol-2-yl]-N-methyl-2-[4-(2-pyridinyl)-phenyl]acetamide methane-sulfonic acid monohydrate, 100 mg and 25 mg (calculated as free base form) tablet cores containing the inventive N-[5-(aminosulfonyl)-4-methyl-1,3-thiazol-2-yl]-N-methyl-2-[4-(2-pyridinyl)-phenyl]acetamide mono methanesulfonic acid monohydrate in an amount of 129 and 32.3 mg respectively were prepared.

### Type and size of the tablet cores

- 100 mg tablet core, diameter 11 mm
- 25 mg tablet core, diameter 7 mm

### Equipments

Blending: Bohle MC5 with glass tube

### Tabletting

- Kilian rotary press with chamber feed shoe
- Tooling 100 mg dose strength: 11 r11 or 12r11 with break score on one site
- Tooling 25 mg dose strength: 7r6

### Analytics

- Dissolution (1000 ml, 0.1 M HCl, paddle apparatus according to USP apparatus 2, paddle speed 50 rounds per minute, n=6)

### Compounds (API, active pharmaceutical ingredient)

API: N-[5-(aminosulfonyl)-4-methyl-1,3-thiazol-2-yl]-N-methyl-2-[4-(2-pyridinyl)-phenyl]acet-amide methane-sulfonic acid monohydrate, wherein at least 65 v/v% N-[5-(Aminosulfonyl)-4-methyl-1,3-thiazol-2-yl]-N-methyl-2-[4-(2-pyridinyl)-phenyl]-acetamide mono methanesulfonic acid monohydrate particles fall in a particle size range of from 2 to 500 micrometer (µm).

G01, G02, G03, G04, G05 are five different tablet test batches of the N-[5-(aminosulfonyl)-4-methyl-1,3-thiazol-2-yl]-N-methyl-2-[4-(2-pyrid inyl)-phenyl]acetamide methane-sulfonic acid monohydrate, wherein the particle size is in a range from 1 to 400 µm with a particle size distribution of d(0.1), d(0.5) and d(0.9) shown by Table 1.

**Table 1: Particle size distribution (PSD) of different batches of N-[5-(aminosulfonyl)-4-methyl-1,3-thiazol-2-yl]-N-methyl-2-[4-(2-pyridinyl)-phenyl]acetamide methane-sulfonic acid monohydrate.**

| **API batch number** | **Trial batch number** | **Particle size distribution** | | | **Specific surface area** |
|---|---|---|---|---|---|
| | | ***d(0.1) [µm]*** | ***d(0.5) [µm]*** | ***d(0.9) [µm]*** | ***[m²*/*g]*** |
| BXR4FLS | G01 | 3 | 51 | 102 | 0.7 |
| BXR3NC1 | G02 | 43 | 118 | 254 | 0.1 |
| NE-023932-A-4-26 crude 1#1 M1 | G03 | 1 | 4 | 11 | 2.4 |
| NE-023932-A-4-27 IPC 1#1 | G04 | 22 | 66 | 143 | 0.2 |
| NE-023932-batch-02-2010 | G05 | 52 | 129 | 257 | 0.1 |

### Preparation of final blends

Five final blends (batches G01 to G05) with the different API batches were prepared. The formulations of the cores (25 mg and 100 mg dose strength, calculated as free base form) are shown in Table 2.

**Table 2:Formulation 25 mg and 100 mg dose strength (calculated as free base form)**

| **Component** | **mg per 25 mg tablet** | **mg per 100 mg tablet** |
|---|---|---|
| N-[5-(aminosulfonyl)-4-methyl-1,3-thiazol-2-yl]-N-methyl-2-[4-(2-pyridinyl)-phenyl]acetamide mono methanesulfonic acid monohydrate | 32.3 (salt) | 129.0 (salt) |
| Microcrystalline cellulose | 60.9 | 243.4 |
| Croscarmellose sodium | 9.8 | 39.0 |
| Mannitol | 20.0 | 80.1 |
| Silica, colloidal anhydrous | 1.3 | 5.0 |
| Magnesium stearate | 0.9 | 3.5 |
| **Sum final blend** | **125.2** | **500.0** |

The final blends were prepared by sieving the raw materials using a 1.0 mm sieve. N-[5-(aminosulfonyl)-4-methyl-1,3-thiazol-2-yl]-N-methyl-2-[4-(2-pyridinyl)-phenyl]-acetamide methane-sulfonic acid monohydrate, i. e. the API, wherein the particle size distribution is in a range from 1 to 400 µm, microcrystalline cellulose, croscarmellose sodium, mannitol and silica colloidal anhydrous were mixed for 30 minutes in a free fall blender. Magnesium stearate was added, and the blends were mixed for additional 5 minutes. No problems were observed during the process.

The bulk properties of batches G01 to G05 are shown in Tables 3 and 4.

**Table 3: Bulk properties of final blends**

| **Parameter** | **G01** | **G02** | **G03** | **G04** | **G05** |
|---|---|---|---|---|---|
| Bulk density [g/ml] | 0.413 | 0.435 | 0.385 | 0.403 | 0.440 |
| Tapped density [g/ml] | 0.510 | 0.526 | 0.563 | 0.500 | 0.550 |
| Flowability [cot] | 1.25 | 1.16 | 0.893 | 1.09 | 1.28 |

A significant difference in bulk density was seen for batch G03. This was the final blend, which contains the micronised API. Therefore, the bulk density was lower compared to the blends, which contain coarser API.

Batch G03 also had the worst flowability of all final blends. This may also be explained by the use of micronised API.

### Tabletting

Final blends from batches G01 to G04 and a part of final blend batch G05 were compressed as a 100 mg dose strength. The other part of the final blend batch G05 was compressed as a 25 mg dose strength.

During compression of the 100 mg dose strength batches G01, G02, G04 and G05, no further problems occurred.

Surprisingly, it was found that the tablet weight was fluctuating during compression of batch G03. Unexpectedly, problems with uniformity of weight occurred. In addition, the tablet mass was sticking to the tools. It was obvious that both problems were caused by use of the micronised API. Therefore, it is advantageous to omit a micronisation step for tabletting, which will result in better handling and less production time.

Compression of the 25 mg dose strength batch G05 was done without problems.

The IPC (in process checks) data of batches G01 - G05 are shown in Tables 4 and 5.

**Table 4: IPC data 100 mg strength**

| **Parameter** | **Nominal value** | **G01** | **G02** | **G03** | **G04** | **G05** |
|---|---|---|---|---|---|---|
| Weight[mg] | 485 - 515 | 498 | 500 | 497 | 506 | 516 |
| Diameter [mm] | 10.8-11.2 | 12.1 | 12.1 | 12.1 | 12.1 | 12.1 |
| Height [mm] | determine | 5.7-5.8 | 5.7 | 5.7-5.8 | 5.7 | 5.6-5.7 |
| Hardness [N] | >50 | 73 | 70 | 67 | 74 | 87 |
| Disintegration [min] | <5 | 20-30 sec | 15-25 sec | 20-30 sec | 20-25 sec | 15-25 sec |

**Table 5: IPC data 25 mg strength**

| **Parameter** | **Nominal value** | **G05** |
|---|---|---|
| Weight[mg] | 122 - 128 | 127 |
| Diameter [mm] | 6.8 - 7.2 | 7.1 |
| Height [mm] | determine | 3.6-3.7 |
| Hardness [N] | >50 | 56 |
| Disintegration [min] | <5 | 20-28 sec |

### Analytics

Tablets or capsules taken orally remain one of the most effective means of treatment available. The effectiveness of such dosage forms relies on the drug dissolving in the fluids of the gastrointestinal tract prior to absorption into the systemic circulation. The rate of dissolution of the tablet or capsule is therefore crucial.

Dissolution: The criteria for dissolution in media with a pH of 1 is Q=75 after 45 minutes.

### Influence of particle size distribution on the dissolution:

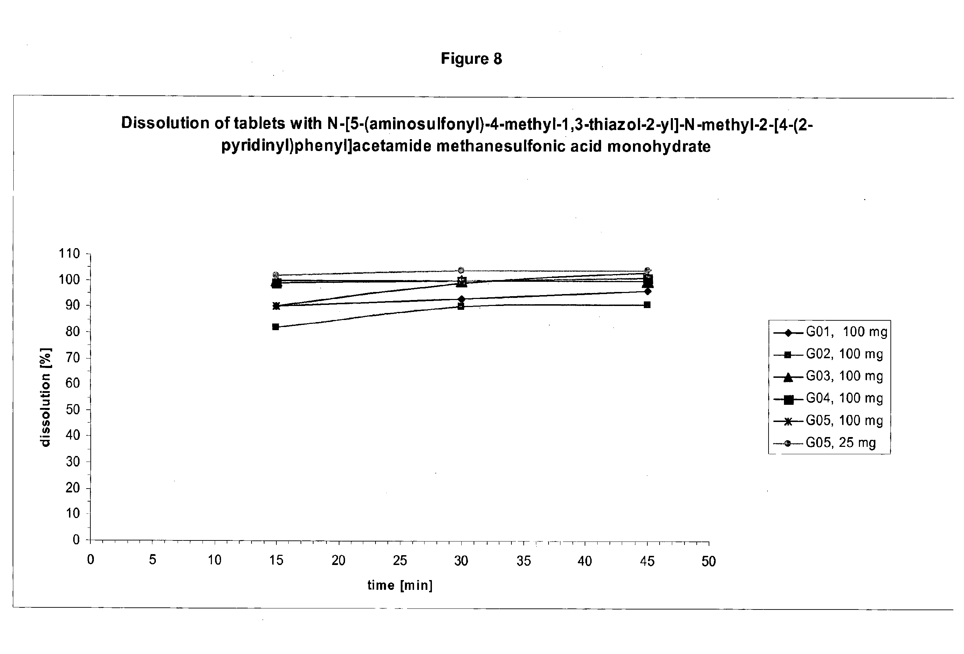

Dissolution of cores from batches G01 to G05 was performed. The dissolution results are shown in Figure 8 and Table 6.

**Table 6: Dissolution results for batches G01 - G05**

| | **15** | | | **30** | | | **45** | | |
|---|---|---|---|---|---|---|---|---|---|
| **Batch** | **Average [%]** | **Min [%]** | **Max [%]** | **Average [%]** | **Min [%]** | **Max [%]** | **Average [%]** | **Min [%]** | **Max [%]** |
| G01 100 mg | 90 | 83 | 97 | 93 | 85 | 99 | 96 | 90 | 99 |
| G02 100 mg | 82 | 80 | 84 | 90 | 88 | 91 | 91 | 90 | 94 |
| G03 100 mg | 100 | 98 | 101 | 100 | 97 | 102 | 100 | 98 | 102 |
| G04 100 mg | 99 | 97 | 100 | 100 | 98 | 102 | 101 | 101 | 102 |
| G05 100 mg | 90 | 87 | 93 | 99 | 98 | 102 | 103 | 102 | 105 |
| G05 25 mg | 102 | 101 | 104 | 104 | 102 | 105 | 104 | 102 | 105 |

The dissolution results of batches G01 - G05 are according to the specification. There is no significant difference in the dissolution of cores, which contains finer or coarser API.

Content Uniformity (CU): Content uniformity of batches G01 to G04 was tested. The results are shown in Table 7.

**Table 7: CU results of batches G01 - G04**

| **Batch** | **Assay mean (%)** | **Standard deviation** | **Acceptance value** |
|---|---|---|---|
| G01 | 98.2 | 1.1917 | 3.2 |
| G02 | 101.2 | 1.2599 | 3.0 |
| G03 | 97.9 | 3.1110 | 8.1 |
| G04 | 103.3 | 1.3640 | 5.1 |

All results comply with Ph.Eur. 2.9.40. Content uniformity of batch G03 is inferior compared to the content uniformity of the other batches. It is obvious that this is related to the problems during compression based on the micronized API.

### CONCLUSIONS

The particle size distribution of the API has an influence on the bulk properties of the final blend and on the tabletting characteristics. Batch G03, which contains the micronized API, has a worse flowability. Therefore, problems with a uniform weight occurred during compression. In addition, sticking problems were observed, and the CU results of the cores are not as good as the results of the cores with non-micronized API. By use of non-micronized API there was no influence of the particle size distribution, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, on uniformity of weight and CU obtained. The results do not show a marked influence of the particle size distribution on the dissolution of the cores as they are all within the predefined ranges.

### Example 3:

Tablet comprising 60 mg of N-[5-(aminosulfonyl)-4-methyl-1,3-thiazol-2-yl]-N-methyl-2-[4-(2-pyridinyl)-phenyl]acetamide (corresponding to 76.9 mg of crystalline N-[5-(aminosulfonyl)-4-methyl-1,3-thiazol-2-yl]-N-methyl-2-[4-(2-pyridinyl)-phenyl]-acetamide mono methanesulfonic acid monohydrate according to the invention), wherein at least 65 v/v% are N-[5-(aminosulfonyl)-4-methyl-1,3-thiazol-2-yl]-N-methyl-2-[4-(2-pyridinyl)-phenyl]acetamide mono methanesulfonic acid monohydrate particles, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g; content of active compound about 59% (based on an unvarnished tablet):

| | |
|---|---|
| crystalline N-[5-(aminosulfonyl)-4-methyl-1,3-thiazol-2-yl]-N-methyl-2-[4-(2-pyridinyl)-phenyl]acetamide methane-sulfonic acid monohydrate | 76.9 mg |
| Avicel PH 101 | 118.0 mg |
| lactose, fine | 40.0 mg |
| Ac-Di-Sol | 20.0 mg |
| polyinylpyrrolidone 25 | 10.0 mg |
| magnesium stearate | 2.0 mg |
| total weight | 266.9 mg |

### Example 4:

Tablet comprising 50 mg of N-[5-(aminosulfonyl)-4-methyl-1,3-thiazol-2-yl]-N-methyl-2-[4-(2-pyridinyl)-phenyl]acetamide (corresponding to 64 mg of crystalline N-[5-(aminosulfonyl)-4-methyl-1,3-thiazol-2-yl]-N-methyl-2-[4-(2-pyridinyl)-phenyl]acetamide mono methanesulfonic acid monohydrate), wherein at least 65 v/v% are N-[5-(aminosulfonyl)-4-methyl-1,3-thiazol-2-yl]-N-methyl-2-[4-(2-pyridinyl)-phenyl]acetamide mono methanesulfonic acid monohydrate particles, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g;
content of active compound about 59% (based on an unvarnished tablet):

| | |
|---|---|
| crystalline N-[5-(aminosulfonyl)-4-methyl-1,3-thiazol-2-yl]-N-methyl-2-[4-(2-pyridinyl)-phenyl]acetamide methane-sulfonic acid monohydrate | 64.0 mg |
| polyinylpyrrolidone 25 | 3.5 mg |
| microcrystalline cellulose | 20.0 mg |
| croscamellose sodium | 10.0 mg |
| magnesium stearate | 0.9 mg |
| optionally HPMC varnish | 3.0 mg |
| total weight | 101.4 mg |

### Example 5:

Crystal structure of N-[5-(aminosulfonyl)-4-methyl-1,3-thiazol-2-yl]-N-methyl-2-[4-(2-pyridinyl)phenyl]acetamide mono methanesulfonic acid monohydrate Formula C₁₉H₂₄N₄O₇S₃, M = 516.62, F(000) = 540,
colorless plate, size 0.02 · 0.13 · 0.15 mm³, triclinic, space group P -1 , Z = 2, a = 9.4908(7)A, b = 9.5545(7) A, c = 14.4137(9) A, α = 86.130(3)°, β = 72.104(3)°, γ = 68.253(4)°, V = 1153.68(15) Å³, D_{calc}. = 1.487 Mg·m⁻³. The crystal was measured on a Nonius KappaCCD diffractometer at 293 K using graphite-monochromated Mo K_{α}-radiation with λ = 0.71073 Å, Θₘₐₓ = 30.065°. Minimal/maximal transmission 0.95/0.99, µ = 0.370 mm⁻¹. The COLLECT suite has been used for data collection and integration. Of 43492 reflections in total, 6761 were independent (merging r = 0.026). From these, 4955 were considered observed (l>3.0σ(l)) and were used to refine 298 parameters. The structure was solved by direct methods using the program SIR92. Least-squares refinement against F was carried out on all non-hydrogen atoms using the program CRYSTALS. R = 0.0313 (observed data), wR = 0.0432 (all data), GOF = 1.0736. Minimal/maximal residual electron density = -0.28/0.33 e Å⁻³. Chebychev polynomial weights were used to complete the refinement.

Table 8 shows single-crystal structure parameters for N-[5-(aminosulfonyl)-4-methyl-1,3-thiazol-2-yl]-N-methyl-2-[4-(2-pyridinyl)phenyl]acetamide mono methanesulfonic acid monohydrate.

**Table 8. Single-crystal structure parameters for N-[5-(aminosulfonyl)-4-methyl-1,3-thiazol-2-yl]-N-methyl-2-[4-(2-pyridinyl)phenyl]acetamide mono methanesulfonic acid monohydrate.**

| **Parameter** | **Value** |
|---|---|
| formula | C₁₉H₂₄N₄O₇S₃ |
| formula weight | 516.62 g/mol |
| Z, calculated density | 2, 1.487 Mg×m⁻³ |
| F(000) | 540 |
| description and size of crystal | Colourless plate, 0.02×0.13×0.15 mm³ |
| absorption coefficient | 0.370 mm⁻¹ |
| min/max transmission | 0.95/0.99 |
| temperature | 293 K |
| radiation (wavelength) | Mo *K*α (λ=0.7103 Å) |
| crystal system, space group | Triclinic, P -1 |
| a | 9.4908 (7) Å |
| b | 9.5545(7) Å |
| c | 14.4137(9) Å |
| α | 86.130(3)° |
| β | 72.104(3)° |
| Y | 68.253(4)° |
| V | 1153.68(15) Å³ |
| min/max Θ | 2.426°/30.065° |
| number of collected reflections | 43492 |
| number of independent reflections | 6761 (merging r=0.026) |
| number of observed reflections | 4955 (l>3.0σ(l)) |
| number of refined parameters | 298 |
| r | 0.0313 (observed data with) |
| rW | 0.0432 (all data) |
| goodness of fit | 1.0736 |
| residual electron density | -0.28/0.33 eÅ⁻³ |

### Example 6:

### Single-dose escalation and pharmacokinetics

An advantage of the tablets according to the invention is that the tablet will have an optimised dissolution rate based on the particle size distribution of the crystalline form of N-[5-(aminosulfonyl)-4-methyl-1,3-thiazol-2-yl]-N-methyl-2-[4-(2-pyridinyl)-phenyl]acetamide mono methanesulfonic acid monohydrate, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, and thus, the drug may be absorbed into the blood stream much faster compared to N-[5-(aminosulfonyl)-4-methyl-1,3-thiazol-2-yl]-N-methyl-2-[4-(2-pyridinyl)-phenyl]acetamide as crystalline base form. Furthermore, the surprising dispersion times obtained with tablets according to the invention are advantageous for swallowable tablets. In a further embodiment, the tablets according to the invention can be presented for dispersion in water.

Therefore, pharmacokinetic studies in human subjects were undertaken following both single and multiple dose administrations of the mesylate monohydrate salt of N-[5-(aminosulfonyl)-4-methyl-1,3-thiazol-2-yl]-N-methyl-2-[4-(2-pyridinyl)phenyl]-acetamide, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g.

Single oral doses of the mesylate monohydrate salt of N-[5-(aminosulfonyl)4-methyl1,3thiazol-2-yl]-N-methyl-2-[4-(2-pyridinyl)phenyl] acetamide using the formulations according to example 2, Table 2, were administered to six volunteers per dose step. The overall shape of the plasma concentrations vs. time profiles were similar across all doses.

There was a rapid and continuous increase of plasma concentrations switching into a period of markedly slower absorption rate and evidence of a plateau effect in exposure. Thereafter, for all doses investigated, there was a decrease of concentrations starting after 4.0 to 4.5 hours post administration. This phase was followed by a phase of prolonged exposure, which was characterised by a long half-life, which is favourable for the treatment of infectious diseases. The mean terminal elimination half-life (t_{1/2z}) ranged between 52 h and 85 h.

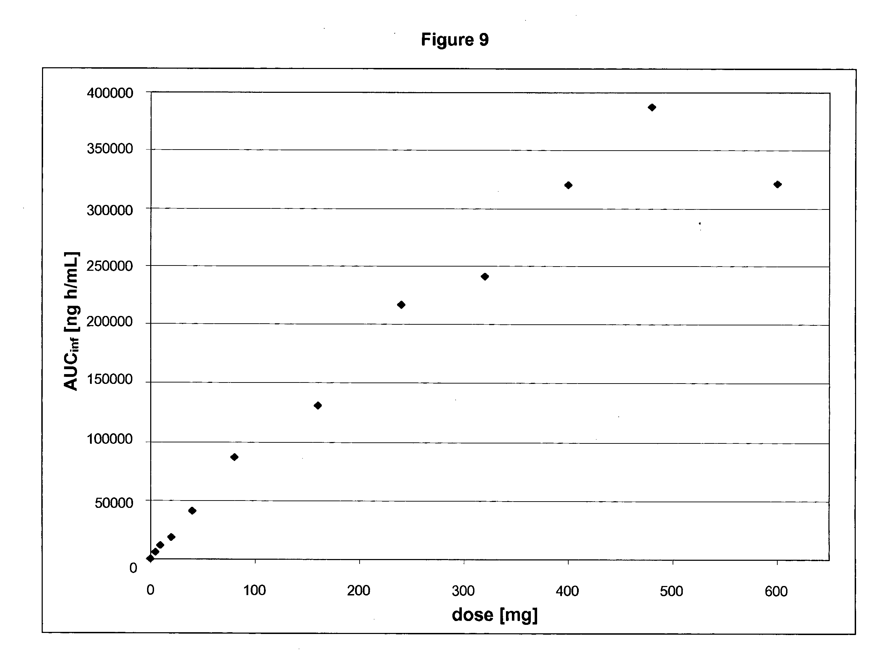

For doses from 5 mg to 480 mg there was a dose-proportional increase in AUC_{∞} (AUC, area under curve); a single dose of 600 mg did not cause any further rise of exposure as shown by AUC_{0-∞} (see Figure 9).

Maximum plasma concentrations were linearly related to doses from 5 mg to 400 mg. At the higher dose up to 600 mg no further increase of exposure was obtained as shown by the both Cₘₐₓ and AUC_{0-∞}. Median tₘₐₓ ranged from 1.5 to 4.25 hours without any obvious relation to dose. A summary of single-dose pharmacokinetic parameters is shown in Table 9.

**Table 9: Pharmacokinetic parameters after ascending single oral doses of mono mesylate monohydrate salt of N-[5-(aminosulfonyl)-4-methyl-1,3-thiazol-2-yl]-N-methyl-2-[4-(2-pyridinyl)phenyl]acetamide in the formulations according to the formulation described on page 20** wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g.

In the following the abbreviations used in Table 9 are defined.

AUC_{0-∞}: Area under the analyte vs. time concentration from time of administration up to infinity, calculated as 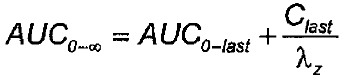 wherein AUC₀₋ₗₐₛₜ is defined as the area under the analyte vs. time concentration up to time of the last qualifiable concentration, calculated by linear up/In down summation and Cₗₐₛₜ is defined as last quantifiable observed analyte concentration. λ_{z} is the apparent terminal elimination rate constant, determined by linear regression of terminal points of In-linear analyte concentration-time curve.
Cₘₐₓ is the maximal observed analyte concentration and tₘₐₓ is the time to reach Cₘₐₓ;
t_{1/2z} is defined as the apparent terminal elimination half-life, calculated as 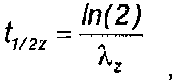 wherein λ_{z} is defined as above.

MRT: Mean Residence Time; calculated AUMC divided by AUC, wherein AUMC is the area under the first moment of the concentration-time curve from zero up to ∞ with extrapolation of the terminal phase and AUC is the area under the concentration-time curve from zero up to ∞ with extrapolation of the terminal phase.

CL/F refers to the clearance after oral administration of a drug and Aₑ refers to the amount of drug excreted in the urine.

**Table 9:**

| **dose [mg]** | **parameter (means; n=6 volunteers/dose)** | | | | | | |
|---|---|---|---|---|---|---|---|
| | AUC₀-_{∞} [ng · h/mL] | Cₘₐₓ [ng/mL] | tₘₐₓ^{a} [h] | t_{1/2z} [h] | MRT [h] | CL/F [L/h] | Aₑ [% of dose] |
| 5 | 5800 | 74 | 4.00 | 80 | 117 | 0.89 | 0 |
| 10 | 11670 | 170 | 1.50 | 85 | 116 | 0.87 | 0 |
| 20 | 18540 | 234 | 2.77 | 76 | 105 | 1.10 | 0 |
| 40 | 40680 | 608 | 3.50 | 72 | 94 | 1.00 | 0 |
| 80, males | 87220 (99790)^{b} | 1306 (1499)^{b} | 2.50 | 74 | 94 | 0.94 | 0.16 |
| 80, females | 96230 (90050)^{b} | 1999 (1853)^{b} | 4.00 | 58 | 77 | 0.85 | 0.31 |
| 160 | 130800 | 2613 | 3.50 | 63 | 83 | 1.28 | 0.15 |
| 240 | 216900 | 3600 | 4.00 | 64 | 82 | 1.15 | 0.21 |
| 320 | 241100 | 4648 | 2.25 | 57 | 70 | 1.47 | 0.16 |
| 400 | 320300 | 6926 | 4.25 | 57 | 64 | 1.31 | 0.15 |
| 480 | 387200 | 6921 | 3.25 | 53 | 72 | 1.33 | 0.26 |
| 600 | 320800 | 6442 | 4.25 | 52 | 65 | 2.00 | 0.09 |

| | | | | | | | |
|---|---|---|---|---|---|---|---|
| ^{a}: for tₘₐₓ the median is given, ^{b}: value normalized to body weight for a 70 kg subject; | | | | | | | |

Based on data for the 80 mg dose, women appeared to exhibit a higher exposure compared to males according to AUC_{∞} and Cₘₐₓ (see Table 9). However, normalization to body weight revealed that this apparent difference could be explained by the lower body weight of the female volunteers compared to males. Therefore, a clinical relevance of any gender-related differences of pharmacokinetics should not be expected.

**Summary of the results of Example 6:** For doses from 5 mg to 400 and 480 mg, respectively, there was a linear, i.e. dose-proportional, increase in AUC_{∞} and Cₘₐₓ with dose; higher doses do not further increase the exposure. The mean terminal elimination half-life (t_{1/2z}) ranged between 52 h and 85 h. No clinical relevant gender-related difference in exposure was detected for a single dose of 80 mg.

### Example 7:

### Multiple-dose escalation and pharmacokinetics

For the three doses of the mesylate monohydrate salt of N-[5-(aminosulfonyl)-4-methyl-1,3-thiazol-2-yl]-N-methyl-2-[4-(2-pyridinyl)phenyl]acetamide in formulation according to example 1, Table 2, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, investigated (5, 25, and 100 mg; once per day oral administration, 20 days), the individual concentration-time curves of N-[5-(aminosulfonyl)-4-methyl-1,3-thiazol-2-yl]Nmethyl-2-[4-(2-pyridinyl)phenyl]acetamide at day 1 (after the first administration) were very similar in their general shape and slope to those profiles obtained in the single dose escalation trial (see Example 6). As for the single dose escalation trial presented in Example 6, there were dose-proportional increases in AUC₀₋₂₄ₕ and Cₘₐₓ at day 1.

During the 20-day treatment with administrations of the mesylate monohydrate salt of N-[5-(aminosulfonyl)-4-methyl-1,3-thiazol-2-yl]-N-methyl-2-[4-(2-pyridinyl)-phenyl]acetamide, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, once daily, the attainment of steady-state conditions was demonstrated by virtually identical minimal or "trough" concentrations of N-[5-(aminosulfonyl)-4-methyl-1,3-thiazol-2-yl]-N-methyl-2-[4-(2-pyridinyl)phenyl]acetamide achieved between days 9 and 13. At steady state, there was a low inter-individual variability of minimal or "trough" concentrations with CVs (coefficient of variations) between 16.7 and 21.7% (day 21). For all doses, the individual and mean concentration-time curves of N-[5-(aminosulfonyl)-4-methyl-1,3-thiazol-2-yl]-N-methyl-2-[4-(2-pyridinyl)phenyl]acetamide at day 21 were very similar in their shape and slope to those profiles obtained at day 1.

Table 10 summarises the steady-state pharmacokinetics of N-[5-(aminosulfonyl)-4-methyl-1,3-thiazol-2-yl]-N-methyl-2-[4-(2-pyridinyl)phenyl]acetamide.

**Table 10: Steady state pharmacokinetic parameters of N-[5-(aminosulfonyl)-4-methyl-1,3-thiazol-2-yl]-N-methyl-2-[4-(2-pyridinyl)phenyl]acetamide at day 21 after daily administrations of 5, 25, or 100 mg of the mesylate monohydrate salt of N-[5-(aminosulfonyl)-4-methyl-1,3-thiazol-2-yl]-N-methyl-2-[4-(2-pyridinyl)phenyl]acetamide in formulation according to example 2, Table 2,** wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, to healthy volunteers (n = 12 per dose).

In the following the abbreviations used in Table 10 are defined.

C_{trough}: measured plasma concentration immediately before dosing at day 21 (at the end of the dosing interval at steady state); AUC_{T}, i.e. the steady state AUC (area under the curve) within the dosing interval of 24 hours, C_{max,ss} refers to the maximal observed analyte concentration at steady state. Cₐᵥ is defined as average plasma concentration during the dosing interval the mono mesylate monohydrate salt of N-[5-(aminosulfonyl)-4-methyl-1,3-thiazol-2-yl]-N-methyl-2-[4-(2-pyridinyl)phenyl]acetamide at steady state, R-AUC refers to the accumulation ratio of the AUC, i.e. AUC_{T} / AUC_{0-24h,day1}, R-Cₘₐₓ refers to accumulation ratio of Cₘₐₓ, i.e. Cₘₐₓ,ₛₛ / C_{max, day1}; t_{1/2z} is defined as above

**Table 10:**

| **dose [mg]** | **C_{trough} [ng/mL]** | **AUC_{T} [ng · h/mL]** | **C_{max,ss} [ng/mL]** | **Cₐᵥ [ng/mL]** | **R-AUC** | **R-Cₘₐₓ** | **t_{1/2z} [h]** |
|---|---|---|---|---|---|---|---|
| 5 | 187 | 5094 | 301 | 213 | 5.2 | 5.0 | 82.6 |
| 25 | 832 | 23430 | 1358 | 977 | 5.3 | 5.3 | 68.6 |
| 100 | 3743 | 108800 | 6358 | 4540 | 5.1 | 5.3 | 59.8 |

For the three doses applied, there was a dose-proportional increase for all measures of exposure at steady state (C_{trough}, AUC_{T}, Cₘₐₓ,ₛₛ, and Cₐᵥ) (see Table 10).

For both AUC and Cₘₐₓ, the accumulation ratio R of all doses applied was very similar being approximately a factor of 5 (see Table 10).

The time to reach Cₘₐₓ,ₛₛ was similar for the three doses (0.5 - 4.5 h). The peak-trough fluctuation at steady-state ranged between 59 and 64%.

Elimination half-life was in the same range as after single-dose application with 82.6 h (5 mg), 68.6 h (25 mg), and 59.8 h (100 mg). The apparent total clearance (CL/F) was estimated to be similar for all doses investigated (0.99 - 1.08 L/h).

**Summary of the results of Example 7:** Under steady-state conditions, an increase in the dose of mesylate monohydrate salt of N-[5-(aminosulfonyl)-4-methyl-1,3-thiazol-2-yl]-N-methyl-2-[4-(2-pyridinyl)phenyl]acetamide in formulation according to example 2, Table 2, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, resulted in a proportional increase in exposure to N-[5-(aminosulfonyl)-4-methyl-1,3-thiazol-2-yl]-N-methyl-2-[4-(2-pyridinyl)phenyl]acetamide. In general, plasma concentrations at steady state are to be expected to be approximately five times higher than after single dose administration of the same dose this being a reflection of the half-life and dosing interval. Inter-individual variability of steady state exposure was quite low as revealed by a low coefficient of variation, e.g. for minimal or "trough" concentrations and peak-trough fluctuations. Rate of elimination and terminal half-lives at steady-state were comparable to the single dose situation.

These results clearly demonstrate that using the mesylate monohydrate salt of N-[5-(aminosulfonyl)-4-methyl-1,3-thiazol-2-yl]-N-methyl-2-[4-(2-pyridinyl)phenyl]-acetamide, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g in the formulations as described above, a once daily dose (or even a less frequent administration) is sufficient for reaching an appropriate plasma concentration for the treatment of viral diseases, e.g. infection by a herpes virus or herpes viruses. In a further human trial it has been shown that the administration of a higher dose of 400 mg to 600 mg and preferably about 500 mg of the mesylate monohydrate salt of the present invention is also sufficient for reaching an appropriate plasma concentration for the treatment of viral diseases, e.g. infection by a herpes virus or herpes viruses.

### Description of figures

- Figure 1: shows the overlay of X-ray powder diffractions obtained after granulation experiments ; from top to bottom:
N-[5-(aminosulfonyl)-4-methyl-1,3-thiazol-2-yl]-N-methyl-2-[4-(2-pyridinyl)phenyl]acetamide mesylate monohydrate
N-[5-(aminosulfonyl)-4-methyl-1,3-thiazol-2-yl]-N-methyl-2-[4-(2-pyridinyl)phenyl]acetamide (free base),
N-[5-(aminosulfonyl)-4-methyl-1,3-thiazol-2-yl]-N-methyl-2-[4-(2-pyridinyl)phenyl]acetamide, granulation of free base using 1 equivalent of methanesulfonic acid according to WO 2006/103011, example 5; lowest curve shows blank granulation matrix.
A 1:1 (weight per weight) mixture of free base and mesylate monohydrate was analysed with X-ray powder diffraction as calibration of the ratio of free base to salt. The ratio was calculated based on integration and shows the granulation mixture having a content of the mesylate monohydrate form of 8 to 12 percent.

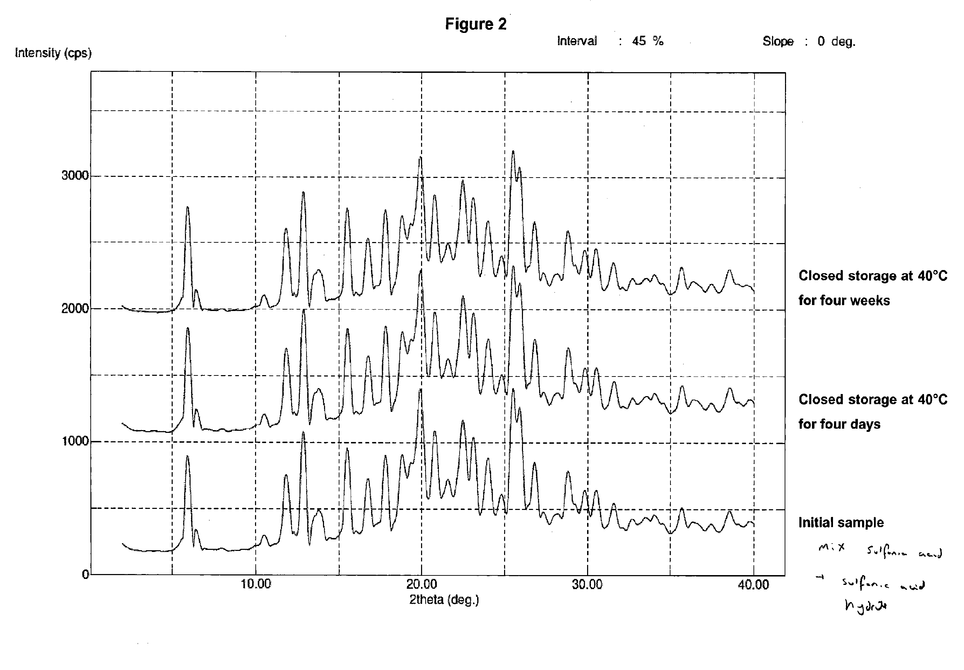

- Figure 2: shows the overlay of X-ray powder diffraction spectra of granulations of a mixture of N-[5-(aminosulfonyl)-4-methyl-1,3-thiazol-2-yl]-N-methyl-2-[4-(2-pyridinyl)phenyl]acetamide mono methanesulfonic acid monohydrate with N-[5-(aminosulfonyl)-4-methyl-1,3-thiazol-2-yl]-N-methyl-2-[4-(2-pyridinyl)phenyl]acetamide mono methane-sulfonic acid after closed storage at 40°C for four weeks (top), after closed storage at 40°C for four days (middle) and of the initial sample, i.e. the mixture of N-[5-(aminosulfonyl)-4-methyl-1,3-thiazol-2-yl]-N-methyl-2-[4-(2-pyridinyl)phenyl]acetamide mono methane-sulfonic acid monohydrate with N-[5-(aminosulfonyl)-4-methyl-1,3-thiazol-2-yl]-N-methyl-2-[4-(2-pyridinyl)phenyl]acetamide mono methanesulfonic acid.
- Figure 3: shows microscope pictures of
A) free base form,
B) crystalline N-[5-(aminosulfonyl)-4-methyl-1,3-thiazol-2-yl]-N-methyl-2-[4-(2-pyridinyl)phenyl]acetamide mono methane-sulfonic acid monohydrate and
C) spontaneous crystallized material after addition of methanesulfonic acid.
- Figure 4: shows the X-ray structure of the N-[5-(aminosulfonyl)-4-methyl-1,3-thiazol-2-yl]-N-methyl-2-[4-(2-pyridinyl)phenyl]acetamide mono methanesulfonic acid monohydrate with indicated hydrogen bridges. It is shown that the nitrogen atom in the pyridinyl ring (right side bottom) is protonated and that a hydrogen bridge is formed between the hydrogen, which protonates the pyridinyl ring nitrogen and one oxygen of the mesylate anion, and another hydrogen bridge is formed between another oxygen of the mesylate anion and the hydrogen of the water molecule while the other hydrogen of the water molecule form a hydrogen bridge with the oxygen of another mesylate anion.
- Figure 5: shows the X-ray powder diffraction spectra of a film-coated tablet with 5 mg N-[5-(aminosulfonyl)-4-methyl-1,3-thiazol-2-yl]-N-methyl-2-[4-(2-pyridinyl)phenyl]acetamide mono methanesulfonic acid monohydrate, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, a placebo tablet and of N-[5-(aminosulfonyl)-4-methyl-1,3-thiazol-2-yl]-N-methyl-2-[4-(2-pyridinyl)
phenyl]acetamide mono methanesulfonic acid monohydrate after 24 months at 25°C.

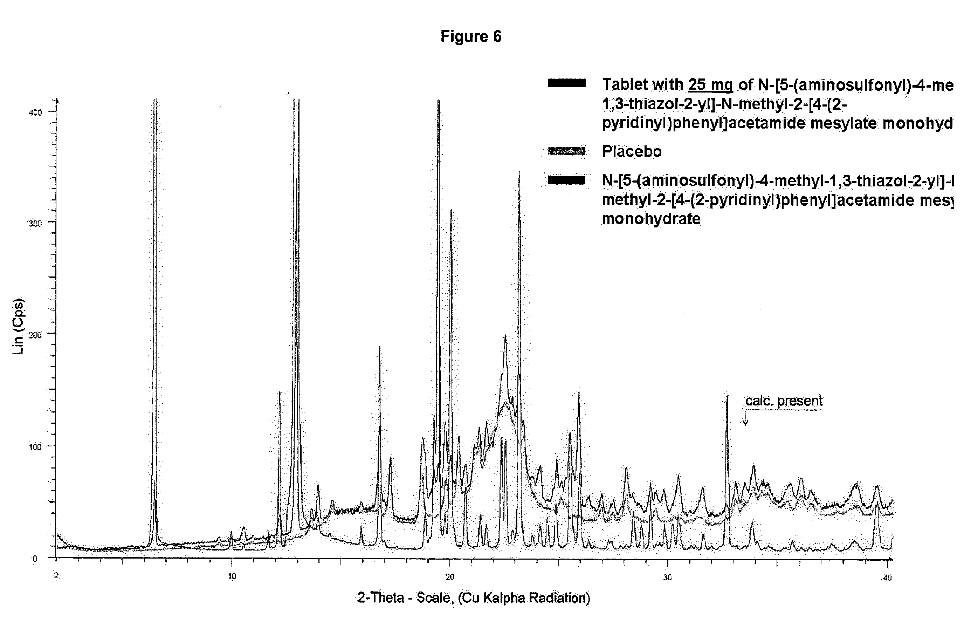

- Figure 6: shows the X-ray powder diffraction spectra of a film-coated tablet with 25 mg N-[5-(aminosulfonyl)-4-methyl-1,3-thiazol-2-yl]-N-methyl-2-[4-(2-pyridinyl)phenyl]acetamide mono methanesulfonic acid monohydrate, wherein the particle size distribution is preferably defined by d(0.1) from 2 to 100 µm, d(0.5) from 30 to 210 µm and d(0.9) from 70 to 400 µm with a specific surface area of the particles less than 1.0 m2/g, and more preferably defined by d(0.1) from 10 to 75 µm, d(0.5) from 100 to 175 µm, d(0.9) from 200 to 350 µm with a specific surface area of the particles less than 0.3 m²/g, a placebo tablet and of N-[5-(aminosulfonyl)-4-methyl-1,3-thiazol-2-yl]-N-methyl-2-[4-(2-pyridinyl)phenyl]acetamide mono methanesulfonic acid monohydrate after 24 months at 25°C

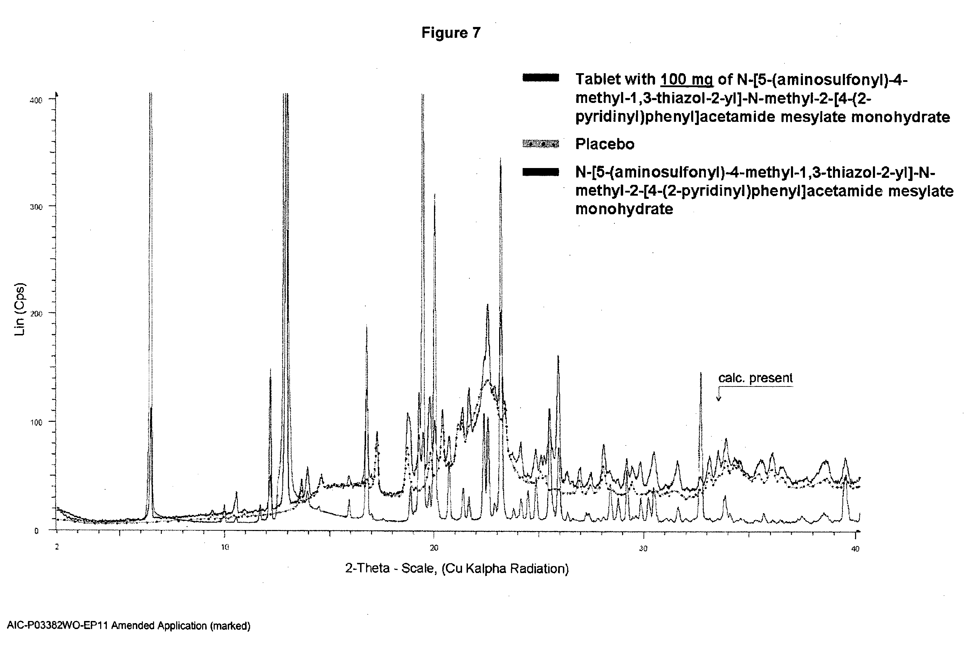

- Figure 7: shows the X-ray powder diffraction spectra of a film-coated tablet with 100 mg N-[5-(aminosulfonyl)-4-methyl-1,3-thiazol-2-yl]-N-methyl-2-[4-(2-pyridinyl)phenyl]acetamide mono methanesulfonic acid monohydrate, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, a placebo tablet and of N-[5-(aminosulfonyl)-4-methyl-1,3-thiazol-2-yl]-N-methyl-2-[4-(2-pyridinyl)phenyl]acetamide mono methanesulfonic acid monohydrate after 24 months at 25°C.
- Figure 8: shows the dissolution curves of the six different tablets according to Table 7.
- Figure 9: shows the relationship between single doses (5 mg - 600 mg) of tablets containing N-[5-(aminosulfonyl)-4-methyl-1,3-thiazol-2-yl]-N-methyl-2-[4-(2-pyridinyl)phenyl]acetamide mono methanesulfonic acid monohydrate, wherein the particle size distribution is preferably defined by d(0.1) from 2 to 100 µm, d(0.5) from 30 to 210 µm and d(0.9) from 70 to 400 µm with a specific surface area of the particles less than 1.0 m²/g, and more preferably defined by d(0.1) from 10 to 75 µm, d(0.5) from 100 to 175 µm, d(0.9) from 200 to 350 µm with a specific surface area of the particles less than 0.3 m²/g, and AUC_{∞}, measured as N-[5-(aminosulfonyl)-4-methyl-1,3-thiazol-2-yl]-N-methyl-2-[4-(2-pyridinyl)phenyl]acetamide.

## Claims

1. Crystalline N-[5-(aminosulfonyl)-4-methyl-1,3-thiazol-2-yl]-N-methyl-2-[4-(2-pyridinyl)-phenyl]acetamide mono methanesulfonic acid monohydrate of the following formula 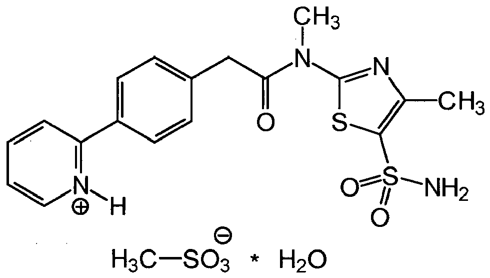 wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a particle size range from 1 to 500 µm, a particle size distribution which is defined by d(0.1) from 2 to 100 µm, d(0.5) from 30 to 210 µm and d(0.9) from 70 to 400 µm and a specific surface area of less than 1.0 m²/g.

2. Crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to claim 1, wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a particle size range from 2 µm to 400 µm.

3. Crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to any previous claim, wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a particle size distribution which is defined by d(0.1) from 10 to 75 µm, d(0.5) from 100 to 175 µm, d(0.9) from 200 to 350 µm.

4. Crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to any previous claim, wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a specific surface area of less than 0.3 m²/g.

5. Crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to any previous claim in combination with acetylsalicylic acid, trifluridine, idoxuridine, foscarnet, cidofovir, ganciclovir, aciclovir, penciclovir, valaciclovir, famciclovir and/or valganciclovir.

6. Crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to any previous claim for use in the treatment and/or prophylaxis of infectious diseases and/or prevention of transmission of an infectious disease.

7. Crystalline N-[5-(aminosulfonyl)-4-methyl-1,3-thiazol-2-yl]-N-methyl-2-[4-(2-pyridinyl)-phenyl]acetamide mono methanesulfonic acid monohydrate according to claim 6, wherein the infectious disease is an infection caused by herpes simplex viruses.

8. Pharmaceutical composition containing crystalline N-[5-(aminosulfonyl)-4-methyl-1,3-thiazol-2-yl]-N-methyl-2-[4-(2-pyridinyl)phenyl]acetamide mono methanesulfonic acid monohydrate particles, wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a particle size range from 1 to 500 µm, a particle size distribution which is defined by d(0.1) from 2 to 100 µm, d(0.5) from 30 to 210 µm and d(0.9) from 70 to 400 µm and a specific surface area of less than 1.0 m²/g together with at least one pharmaceutically acceptable carrier, excipient, solvent and/or diluent.

9. Pharmaceutical composition according to claim 8, wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a particle size range from 2 µm to 400 µm.

10. Pharmaceutical composition according claim 8 or 9, wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a particle size distribution which is defined by d(0.1) from 10 to 75 µm, d(0.5) from 100 to 175 µm, d(0.9) from 200 to 350 µm.

11. Pharmaceutical composition according to any one of claims 8 - 10, wherein the N-[5-(aminosulfonyl)-4-methyl-1,3-thiazol-2-yl]-N-methyl-2-[4-(2-pyridinyl)-phenyl]acetamide mono methanesulfonic acid monohydrate particles have a specific surface area of less than 0.3 m²/g.

12. Pharmaceutical composition according to any one of claims 8 - 11 in combination with acetylsalicylic acid, trifluridine, idoxuridine, foscarnet, cidofovir, ganciclovir, aciclovir, penciclovir, valaciclovir, famciclovir and/or valganciclovir.

13. Pharmaceutical composition according to any one of claims 8 - 11 for use in the treatment and/or prophylaxis of infectious diseases and/or prevention of transmission of an infectious disease.

14. Pharmaceutical composition for use according to claim 13, wherein the infectious disease is a herpes simplex virus infection.

15. Pharmaceutical composition for use according to claim 13 or 14, wherein the pharmaceutical composition is to be administered thrice daily, twice daily, once daily, thrice weekly, twice weekly, or once weekly.

## Patentansprüche

1. Kristallines N-[5-(Aminosulfonyl)-4-methyl-1,3-thiazol-2-yl]-N-methyl-2-[4-(2-pyridinyl)-phenyl]acetamidmonomethansulfonsäuremonohydrat der folgenden Formel 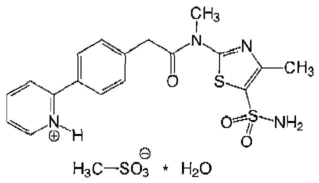 wobei die Partikel aus N-[5-(Aminosulfonyl)-4-methyl-1,3-thiazol-2-yl]-N-methyl-2-[4-(2-pyridinyl)-phenyl]acetamidmonomethansulfonsäuremonohydrat einen Partikelgrößenbereich von 1 bis 500 µm, eine Verteilung der Partikelgröße definiert durch d(0.1) von 2 bis 100 µm, d(0.5) von 30 bis 210 µm und d(0.9) von 70 bis 400 µm und eine spezifische Oberfläche von weniger als 1,0 m²/g aufweisen.

2. Kristallines N-[5-(Aminosulfonyl)-4-methyl-1,3-thiazol-2-yl]-N-methyl-2-[4-(2-pyridinyl)-phenyl]acetamidmonomethansulfonsäuremonohydrat gemäß Anspruch 1, wobei die Partikel aus N-[5-(Aminosulfonyl)-4-methyl-1,3-thiazol-2-yl]-N-methyl-2-[4-(2-pyridinyl)-phenyl]acetamidmonomethansulfonsäuremonohydrat einen Partikelgrößenbereich von 2 µm bis 400 µm aufweisen.

3. Kristallines N-[5-(Aminosulfonyl)-4-methyl-1,3-thiazol-2-yl]-N-methyl-2-[4-(2-pyridinyl)-phenyl]acetamidmonomethansulfonsäuremonohydrat gemäß eines vorherigen Anspruches, wobei die Partikel aus N-[5-(Aminosulfonyl)-4-methyl-1,3-thiazol-2-yl]-N-methyl-2-[4-(2-pyridinyl)-phenyl]acetamid-monomethansulfonsäuremonohydrat eine Verteilung der Partikelgröße definiert durch d(0.1) von 10 bis 75 µm, d(0.5) von 100 bis 175 µm, d(0.9) von 200 bis 350 µm aufweisen.

4. Kristallines N-[5-(Aminosulfonyl)-4-methyl-1,3-thiazol-2-yl]-N-methyl-2-[4-(2-pyridinyl)-phenyl]acetamidmonomethansulfonsäuremonohydrat gemäß eines vorherigen Anspruches, wobei die Partikel aus N-[5-(Aminosulfonyl)-4-methyl-1,3-thiazol-2-yl]-N-methyl-2-[4-(2-pyridinyl)-phenyl]acetamidmono-methansulfonsäuremonohydrat eine spezifische Oberfläche von weniger als 0,3 m²/g aufweisen.

5. Kristallines N-[5-(Aminosulfonyl)-4-methyl-1,3-thiazol-2-yl]-N-methyl-2-[4-(2-pyridinyl)-phenyl]acetamidmonomethansulfonsäuromonohydrat gemäß eines vorherigen Anspruches in Kombination mit Acetylsalicylsäure, Trifluridin, Idoxuridin, Foscarnet, Cidofovir, Ganciclovir, Aciclovir, Penciclovir, Valaciclovir, Famciclovir und/oder Valganciclovir.

6. Kristallines N-[5-(Aminosulfonyl)-4-methyl-1,3-thiazol-2-yl]-N-methyl-2-[4-(2-pyridinyl)-phenyl]acetamidmonomethansulfonsäuremonohydrat gemäß eines vorherigen Anspruches zur Verwendung bei der Behandlung und/oder Prophylaxe von Infektionskrankheiten und/oder zur Vorbeugung der Übertragung einer Infektionskrankheit.

7. Kristallines N-[5-(Aminosulfonyl)-4-methyl-1,3-thiazol-2-yl]-N-methyl-2-[4-(2-pyridinyl)-phenyl]acetamidmonomethansulfonsäuremonohydrat gemäß Anspruch 6, wobei es sich bei der Infektionskrankheit um eine durch Herpes-simplex-Viren ausgelöste Infektion handelt.

8. Pharmazeutische Zusammensetzung enthaltend Partikel aus kristallinem N-[5-(Aminosulfonyl)-4-methyl-1,3-thiazol-2-yl]-N-methyl-2-[4-(2-pyridinyl)phenyl]acetamidmonomethansulfonsäuremonohydrat, wobei die Partikel aus N-[5-(Aminosulfonyl)-4-methyl-1,3-thiazol-2-yl]-N-methyl-2-[4-(2-pyridinyl)-phenyl]acetamidmonomethansulfonsäuremonohydrat einen Partikelgrößenbereich von 1 bis 500 µm, eine Verteilung der Partikelgröße definiert durch d(0.1) von 2 bis 100 µm, d(0.5) von 30 bis 210 µm und d(0.9) von 70 bis 400 µm und eine spezifische Oberfläche von weniger als 1,0 m²/g aufweisen zusammen mit mindestens einem pharmazeutisch verträglichen Träger, Hilfsstoff, Lösungsmittel und/oder Verdünnungsmittel.

9. Pharmazeutische Zusammensetzung gemäß Anspruch 8, wobei die Partikel aus N-[5-(Aminosulfonyl)-4-methyl-1,3-thiazol-2-yl]-N-methyl-2-[4-(2-pyridinyl)-phenyl]acetamidmonomethansulfonsäuremonohydrat einen Partikelgrößenbereich von 2 µm bis 400 µm aufweisen.

10. Pharmazeutische Zusammensetzung gemäß Anspruch 8 oder 9, wobei die Partikel aus N-[5-(Aminosulfonyl)-4-methyl-1,3-thiazol-2-yl]-N-methyl-2-[4-(2-pyridinyl)-phenyl]acetamidmonomethansulfonsäuremonohydrat eine Verteilung der Partikelgröße definiert durch d(0.1) von 10 bis 75 µm, d(0.5) von 100 bis 175 µm, d(0.9) von 200 bis 350 µm aufweisen.

11. Pharmazeutische Zusammensetzung gemäß einem der Ansprüche 8 - 10, wobei die Partikel aus N-[5-(Aminosulfonyl)-4-methyl-1,3-thiazol-2-yl]-N-methyl-2-[4-(2-pyridinyl)-phenyl]acetamidmonomethansulfonsäuremonohydrat eine spezifische Oberfläche von weniger als 0,3 m²/g aufweisen.

12. Pharmazeutische Zusammensetzung gemäß einem der Ansprüche 8 - 11 in Kombination mit Acetylsalicylsäure, Trifluridin, Idoxuridin, Foscarnet, Cidofovir, Ganciclovir, Aciclovir, Penciclovir, Valaciclovir, Famciclovir und/oder Valganciclovir.

13. Pharmazeutische Zusammensetzung gemäß einem der Ansprüche 8 - 11 zur Verwendung bei der Behandlung und/oder Prophylaxe von Infektionskrankheiten und/oder zur Vorbeugung der Übertragung einer Infektionskrankheit.

14. Pharmazeutische Zusammensetzung zur Verwendung gemäß Anspruch 13, wobei es sich bei der Infektionskrankheit um eine Herpes-simplex-Virusinfektion handelt.

15. Pharmazeutische Zusammensetzung zur Verwendung gemäß Anspruch 13 oder 14, wobei die pharmazeutische Zusammensetzung dreimal täglich, zweimal täglich, einmal täglich, dreimal wöchentlich, zweimal wöchentlich, oder einmal wöchentlich zu verabreichen ist.

## Revendications

1. Cristallin N-[5-(aminosulfonyl)-4-méthyl-1,3-thiazol-2-yl]-N-méthyl-2-[4-(2-pyridinyl)phényl]acétamide mono acide méthanesulfonique monohydrate de formule suivante 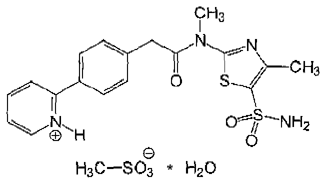 dans lequel les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une plage de la taille de particule de 1 à 500 µm, une distribution de la taille de particule qui est définie par d(0.1) de 2 à 100 µm, d(0.5) de 30 à 210 µm et d(0.9) de 70 à 400 µm et une surface spécifique de moins de 1.0 m²/g.

2. Cristallin N-[5-(aminosulfonyl)-4-méthyl-1,3-thiazol-2-yl]-N-méthyl-2-[4-(2-pyridinyl)phényl]acétamide mono acide méthanesulfonique monohydrate selon la revendication 1, dans lequel les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une plage de la taille de particule de 2 µm à 400 µm.

3. Cristallin N-[5-(aminosulfonyl)-4.-méthyl-1,3-thiazol-2-yl]-N-méthyl-2-[4-(2-pyridinyl)phényl]acétamide mono acide méthanesulfonique monohydrate selon n'importe quelle revendication précédente, dans lequel les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une distribution de la taille de particule qui est définie par d(0.1) de 10 à 75 µm, d(0.5) de 100 à 175 µm, d(0.9) de 200 à 350 µm.

4. Cristallin N-[5-(aminosulfonyl)-4-méthyl-1,3-thiazol-2-yl]-N-méthyl-2-[4-(2-pyridinyl)phényl]acétamide mono acide méthanesulfonique monohydrate selon n'importe quelle revendication précédente, dans lequel les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une surface spécifique de moins de 0.3 m²/g.

5. Cristallin N-[5-(aminosulfonyl)-4-méthyl-1,3-thiazol-2-yl]-N-méthyl-2-[4-(2-pyridinyl)phényl]acétamide mono acide méthanesulfonique monohydrate selon n'importe quelle revendication précédente en combinaison avec de l'acide acétylsalicylique, de la trifluridine, de l'idoxuridine, du foscarnet, du ciclofovir, du ganciclovir, de l'aciclovir, du penciclovir, du valaciclovir, du famciclovir et/ou du valganciclovir.

6. Cristallin N-[5-(aminosulfonyl)-4-méthyl-1,3-thiazol-2-yl]-N-méthyl-2-[4-(2-pyridinyl)phényl]acétamide mono acide méthanesulfonique monohydrate selon n'importe quelle revendication précédente destiné à être utilisé dans le traitement et/ou la prophylaxie des maladies infectieuses et/ou la prévention de la transmission d'une maladie infectieuse.

7. Cristallin N-[5-(aminosulfonyl)-4-méthyl-1,3-thiazol-2-yl]-N-méthyl-2-[4-(2-pyridinyl)phényl]acétamide mono acide méthanesulfonique monohydrate selon la revendication 6, dans lequel la maladie infectieuse est une infection provoquée par les virus de l'herpès simplex.

8. Composition pharmaceutique contenant les particules du cristallin N-[5-(aminosulfonyl)-4-méthyl-1,3-thiazol-2-yl]-N-méthyl-2-[4-(2-pyridinyl)phényl]acétamide mono acide méthanesulfonique monohydrate, dans laquelle les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une plage de la taille de particule de 1 à 500 µm, une distribution de la taille de particule qui est définie par d(0.1) de 2 à 100 µm, d(0.5) de 30 à 210 µm et d(0.9) de 70 à 400 µm et une surface spécifique de moins de 1.0 m²/g en combinaison avec au moins avec un véhicule, excipient, solvant et/ou diluant pharmaceutiquement acceptable.

9. Composition pharmaceutique selon la revendication 8, dans laquelle les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une plage de la taille de particule de 2 µm à 400 µm.

10. Composition pharmaceutique selon la revendication 8 ou 9, dans laquelle les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une distribution de la taille de particule qui est définie par d(0.1) de 10 à 75 µm, d(0.5) de 100 à 175 µm, d(0.9) de 200 à 350 µm.

11. Composition pharmaceutique selon n'importe quelle des revendications 8 - 10, dans laquelle les particules du N-[5-(aminosulfonyl)-4-méthyl-1,3-thiazol-2-yl]-N-méthyl-2-[4-(2-pyridinyl)phényl]acétamide mono acide méthanesulfonique monohydrate ont une surface spécifique de moins de 0.3 m²/g.

12. Composition pharmaceutique selon n'importe quelle des revendications 8 - 11 en combinaison avec de l'acide acétylsalicylique, de la trifluridine, de l'idoxuridine, du foscarnet, du ciclofovir, du ganciclovir, de l'aciclovir, du penciclovir, du valaciclovir, du famciclovir et/ou du valganciclovir.

13. Composition pharmaceutique selon n'importe quelle des revendications 8 - 11 destinée à être utilisée dans le traitement et/ou la prophylaxie des maladies infectieuses et/ou la prévention de la transmission d'une maladie infectieuse.

14. Composition pharmaceutique destinée à être utilisée selon la revendication 13, dans laquelle la maladie infectieuse est une infection au virus de l'herpès simplex.

15. Composition pharmaceutique destinée à être utilisée selon la revendication 13 ou 14, dans laquelle la composition pharmaceutique doit être administrée trois fois par jour, deux fois par jour, une fois par jour, trois fois par semaine, deux fois par semaine, ou une fois par semaine.
